# EUROPEAN PATENT SPECIFICATION

(11) **EP 2 636 735 B1**
(45) Date of publication and mention of the grant of the patent: **04.12.2019**
(21) Application number: 11832651.1
(22) Date of filing: 13.10.2011
(51) Int. Cl.: C12N 15/09, A61K 31/711, A61K 35/76, A61K 39/145, A61K 48/00, A61P 31/16, C12N 7/04

(54) **RECOMBINANT VACCINIA VIRUS HAVING HEMAGGLUTININ PROTEIN GENES DERIVED FROM NOVEL INFLUENZA VIRUSES**
REKOMBINANTES VACCINIA-VIRUS MIT VON NEUEN INFLUENZAVIREN ABGELEITETEN HÄMAGGLUTININPROTEINEN
VIRUS RECOMBINANT DE LA VACCINE AYANT UN GÈNE DE PROTÉINE D'HÉMAGGLUTININE DÉRIVÉE D'UN NOUVEAU TYPE DE VIRUS DE LA GRIPPE

(30) Priority: 15.10.2010 JP 2010233064
(43) Date of publication of application: 11.09.2013
(73) Proprietor: Tokyo Metropolitan Institute of Medical Science, Tokyo 156-8506 (JP); KM Biologics Co., Ltd., Kumamoto 860-8568 (JP); National University Corporation Hokkaido University, Sapporo-shi, Hokkaido 060-0808 (JP)
(72) Inventor: KOHARA, Michinori, Tokyo 156-8506 (JP); YASUI, Fumihiko, Tokyo 156-8506 (JP); MURAKAMI, Toshio, Kikuchi-shi Kumamoto 869-1298 (JP); KIDA, Hiroshi, Hokkaido 060-0808 (JP); SAKODA, Yoshihiro, Hokkaido 060-0808 (JP)
(74) Representative: J A Kemp LLP
(86) International application number: PCT/JP2011/074086
(87) International publication number: WO 2012/050229

(56) References cited:
- EP-A1- 1 925 318
- WO-A1-2006/038742
- WO-A1-2009/068992
- WO-A1-2009/110644
- JP-A- 6 237 773
- JP-A- 2010 510 281
- A. ENDO ET AL: "Homotypic and heterotypic protection against influenza virus infection in mice by recombinant vaccinia virus expressing the haemagglutinin or nucleoprotein gene of influenza virus", JOURNAL OF GENERAL VIROLOGY, vol. 72, no. 3, 1 March 1991 (1991-03-01), pages 699-703, XP055086980, ISSN: 0022-1317, DOI: 10.1099/0022-1317-72-3-699
- MURAKAMI S ET AL: "Cross-clade protective immunity of H5N1 influenza vaccines in a mouse model", VACCINE, ELSEVIER LTD, GB, vol. 26, no. 50, 25 November 2008 (2008-11-25), pages 6398-6404, XP025658437, ISSN: 0264-410X, DOI: 10.1016/J.VACCINE.2008.08.053 [retrieved on 2008-09-17]
- J. MAYRHOFER ET AL: "Nonreplicating Vaccinia Virus Vectors Expressing the H5 Influenza Virus Hemagglutinin Produced in Modified Vero Cells Induce Robust Protection", JOURNAL OF VIROLOGY, vol. 83, no. 10, 11 March 2009 (2009-03-11), pages 5192-5203, XP055178179, ISSN: 0022-538X, DOI: 10.1128/JVI.02081-08
- KITABATAKE M. ET AL.: 'SARS-CoV spike protein-expressing recombinant vaccinia virus efficiently induces neutralizing antibodies in rabbits pre-immunized with vaccinia virus.' VACCINE vol. 25, 2007, pages 630 - 637, XP005798920
- KENNER J. ET AL.: 'LC16m8: An attenuated smallpox vaccine' VACCINE vol. 24, 2006, pages 7009 - 7022, XP028011427
- DATABASE GENBANK [Online] 01 May 2008 SMITH C.: 'Definition: Influenza A virus (A/Indonesia/5/05(H5N1)) segment 4 hemagglutinin (HA) gene, complete cds.', XP003032702 Retrieved from NCBI Database accession no. EF541394
- DATABASE GENBANK [Online] 01 December 2008 HOFFMANN E. ET AL.: 'Definition: Influenza A virus (St Jude H5N1 influenza seed virus 163222) hemagglutinin (HA) gene, complete cds.', XP003032703 Retrieved from NCBI Database accession no. DQ659327
- DATABASE GENBANK [Online] 02 April 2006 SHU Y. ET AL.: 'Definition: Influenza A virus (A/Anhui/1/2005(H5N1)) hemagglutinin (HA) gene, complete cds.', XP003032704 Retrieved from NCBI Database accession no. DQ371928
- BREATHNACH C.C. ET AL.: 'Use of recombinant modified vaccinia Ankara viral vectors for equine influenza vaccination.' VET. IMMUNOL. IMMUNOPATHOL. vol. 98, no. 3-4, 2004, pages 127 - 136, XP002450265
- ENDO A. ET AL.: 'Homotypic and heterotypic protection against influenza virus infection in mice by recombinant vaccinia virus expressing the haemagglutinin or nucleoprotein gene of influenza virus.' J. GEN. VIROL. vol. 72, 1991, pages 699 - 703, XP055086980
- DATABASE GENBANK [Online] 01 June 2009 SHU B. ET AL.: 'Definition: Influenza A virus (A/California/07/2009(HlNl)) segment 4 hemagglutinin (HA) gene, complete cds.', XP003032705 Retrieved from NCBI Database accession no. FJ969540
- KENNER J ET AL: "LC16m8: An attenuated smallpox vaccine", VACCINE, ELSEVIER LTD, GB, vol. 24, no. 47-48, 17 November 2006 (2006-11-17), pages 7009-7022, XP028011427, ISSN: 0264-410X, DOI: 10.1016/J.VACCINE.2006.03.087 [retrieved on 2006-11-17]

## Description

### TECHNICAL FIELD

The present invention relates to prophylactic agents and therapeutic agents for highly pathogenic H5N1 avian influenza virus (H5N1 HPAIV) infection and pandemic H1N1 influenza virus (H1N1 (2009) pdm) infection that are causative of onset of symptoms of novel influenza. More particularly, the present invention relates to recombinant vaccinia viruses that can express hemagglutinin (HA) protein genes of H5N1 HPAIV and H1N1(2009) pdm, and to a prophylactic agent and a therapeutic agent for novel influenza comprising said recombinant vaccinia virus.

### BACKGROUND ART

A patient infected with highly pathogenic H5N1 avian influenza virus (H5N1 HPAIV) was first reported in 1997, and after that the number of infected patients expanded around China, Southeast Asia and the Middle East. So far, 500 patients were taken to the hospitals and treated in high care units but 296 patients, i.e., about 60%, died. Currently, H5N1 HPAIV vaccines, i.e., inactivated whole-virus vaccines of viruses infecting/grown in embryonated chicken eggs are produced and stockpiled in anticipation of an epidemic strain as prepandemic vaccines for four clades (clades 1, 2.1, 2.2 and 2.3). H5N1 HPAIV infection of embryonated chicken eggs, however, is highly deadly and low in virus yield and thus has disadvantages such as difficulty in bulk preparation. Moreover, since the storage life is three years, products are exceeding their storage life sequentially from those produced in 2006. Prepandemic H5N1 vaccine was not produced in fiscal 2009 due to the H1N1(2009) pdm pandemic. In addition, since vaccines are produced just in anticipation of epidemic strain, there are numbers of unclear points as to whether or not the vaccines can exert vaccine effects against the actual epidemic strains.

Meanwhile, the pandemic H1N1 influenza virus (H1N1(2009) pdm) that seems to be originated in Mexico in April, 2009 greatly differs from the past seasonal influenza viruses in terms of antigenicity and thus infection rapidly spread around the world since large number people did not have immunity to this virus. H1N1(2009) pdm vaccine is an inactivated split vaccine that is produced by the same method as the seasonal influenza vaccine. Since this split vaccine is also an inactivated vaccine, it does not necessarily exert vaccine effect against the epidemic strain.

Currently, Tamiflu and Relenza that inhibit the action of neuraminidase (NA) that is important to budding of influenza viruses are known as influenza therapeutic agents, but their effects are limited since, for example, it is important to administer them within 48 hours following the onset of symptoms. Intravenous agents of NA inhibitors and polymerase inhibitors for influenza viruses are also under development but they are yet at the clinical trial stage as of now.

Under the current situation, there is a strong demand for establishment of a prophylactic agent and a therapeutic agent that can exert long-term effect against a wide range of influenza viruses whose epidemics are hard to predict.

Prepandemic H5N1 HPAIV vaccines produced/stockpiled in Japan have been subjected to three types of clinical trials, namely, "safety test" conducted on 6000 people from 2008 to 2009, " initial vaccination test" conducted on 200 people, and "booster vaccination test" conducted on 200 people who had already underwent initial vaccination. In regard to safety, it was found to be within the scope of the assumption. On the other hand, although cross-reactivity was confirmed in the "booster vaccination test" where subjects initially vaccinated with "Vietnam strain" H5N1 vaccine were given different vaccine strains, i.e., Indonesia strain or Anhui strain, induction of neutralizing antibodies against different virus strains was reported to be insufficient in the "initial vaccination test" where two doses of same vaccines were administered at a 3-week interval. Moreover, report of an experiment on protection against infection using mouse models also says that cross-reactivity was particularly low between clades 2.2 and 2.3 of the subtype among these four types of vaccines prepared by the same procedure in laboratory level, and fatality was not completely prevented (Muarakami S. et al., Cross-clade protective immunity of H5N1 influenza vaccines in a mouse model., Vaccine, vol. 26(50), p. 6398-6404, 2008). Endo et al. (J. General Virology 72(Pt 3)(3): 699-703) describes homotypic and heterotypic protection against influenza virus infection in mice by recombinant vaccinia virus expressing the haemoagglutinin or nucleoprotein of influenza virus. EP 1 925 318 describes a recombinant modified vaccinia virus Ankara (MVA) comprising a heterologous nucleic acid from influenza A virus class H5 antigen. Mayrhofer et al. (J. Virology 83(10): 5192-5203) describes the production of nonreplicating vaccinia virus vectors expressing the H5 influenza virus hemagglutinin.

### SUMMARY OF THE INVENTION

The objective of the present invention is to provide a therapeutic agent and a prophylactic agent comprising a recombinant vaccinia virus that is effective in inhibiting the onset of symptoms caused by infection with novel influenza viruses including H5N1 HPAIV

The present inventors have underwent further research based on the above-described results from analyses and reviews of the novel influenza virus infection, and as a result of which came round to the idea that the use of a recombinant vaccinia vaccine that brings about strong immune activation can lead to a potent method for preventing infection with a novel influenza virus. Furthermore, in order to solve the above-described problems, the present inventors have gone through intensive study. As a result, they succeeded in preparing a recombinant vaccinia virus that was effective in inhibiting the onset of symptoms caused by H5N1 HPAIV and H1N1(2009) pdm infections. In particular, a H5N1 HPAIV recombinant vaccinia virus was confirmed to prevent the onset of symptoms against challenge infection with a strain of a different clade at a single-dose vaccination, thereby accomplishing the present invention.
That is to say, the present invention is as follows.
(1) A recombinant vaccinia virus LC16m8 strain, comprising (i) DNA having the nucleotide sequence represented by SEQ ID NO:3 and coding for HA protein derived from clade 2.2 of H5N1 HPAIV; or (ii) DNA having the nucleotide sequence represented by SEQ ID NO:14 and coding for HA protein derived from clade 2.3 of H5N1 HPAIV.
   Furthermore, an example of the expression promoter contained in the recombinant vaccinia virus of the present invention includes a hybrid promoter. Specific examples of the hybrid promoter include those having either DNA (a) or (b) below:
   (a) DNA having the nucleotide sequence represented by SEQ ID NO:6; or
   (b) DNA that hybridizes to DNA having a nucleotide sequence complementary to the nucleotide sequence represented by SEQ ID NO:6 under stringent conditions and that has a promoter activity.
(2) A pharmaceutical composition comprising the recombinant vaccinia virus according to (1) above.

The pharmaceutical composition may be used as a prophylactic agent and/or a therapeutic agent for novel influenza, specifically, as a prophylactic agent and/or a therapeutic agent for highly pathogenic H5N1 avian influenza.

### BRIEF DESCRIPTION OF THE DRAWINGS

Figure 1 is a diagram showing the gene structure of a plasmid used for preparing a recombinant vaccinia virus having HA protein gene of a novel influenza virus. In the diagram, "Influenza HA" represents HA protein gene of each novel influenza virus (cDNAs: SEQ ID NOS:12, 13, 3, 14 and 15) while "Subtype H5" and "Subtype H1" represent H5N1 HPAIV and H1N1(2009) pdm, respectively (the same applies to other figures).
Figure 2 is a diagram showing the positions and names of the primers used for confirming the introduction of HA protein gene by PCR.
Figure 3 shows pictures of agarose gel electrophoresis showing the results of confirming the introduction of HA protein gene by PCR.
Figure 4 shows pictures of PVDF membranes showing the results of confirming the expression of HA protein gene by Western blotting.
Figure 5 is a diagram showing a method of confirming humoral immune-inducing capacity of recombinant vaccinia virus having HA protein gene of H5N1 HPAIV or H1N1(2009) pdm.

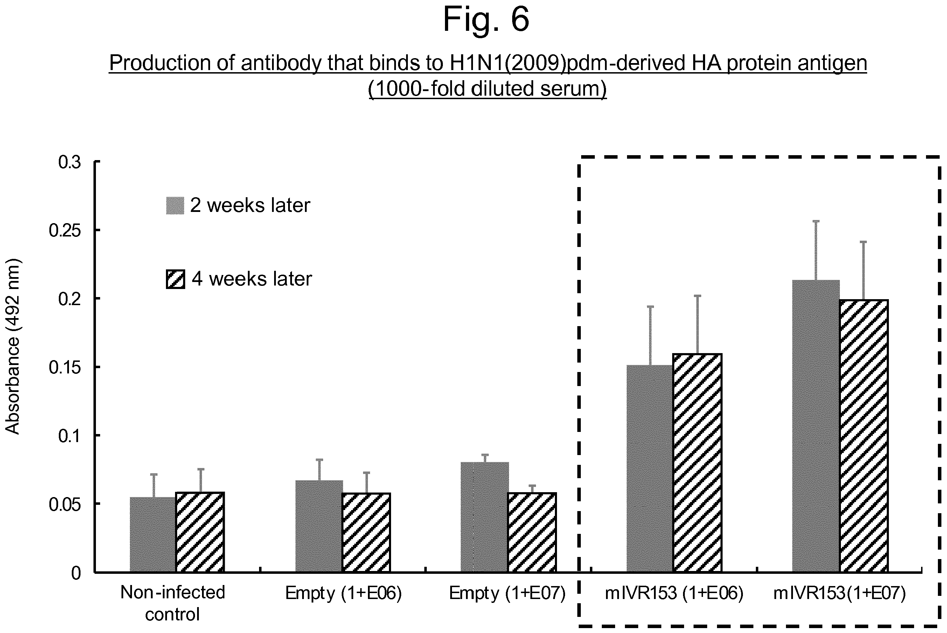

Figure 6 is a diagram showing the results from determination of the effect of humoral immune-inducing capacity (antibody titer) of the recombinant vaccinia virus, as a vaccine, having H1N1(2009) pdm-derived HA protein gene by ELISA. In the figure, "Empty (1+E06)" represents the case where RVV-Empty is inoculated at 1 x 10⁶ PFU, "Empty (1+E07)" represents the case where RVV-Empty is inoculated at 1 x 10⁷ PFU, "mIVR153 (1+E06)" represents the case where RVV-Flu HA (H1-mIVR153) is inoculated at 1 x 10⁶ PFU, and "mIVR153 (1+E07)" represents the case where RVV-Flu HA (H1-mIVR153) is inoculated at 1 x 10⁷ PFU.
Figure 7 is a diagram showing the results from determination of the effect of humoral immune-inducing capacity (antibody titer) of the recombinant vaccinia virus, as a vaccine, having H5N1 HPAIV-derived HA protein gene by ELISA. In the figure, "RVV-mC12.2" and "RVV-mC12.3" represent "RVV-Flu HA (H5-mCl2.2)" and "RVV-Flu HA (H5-mC12.3)", respectively, while "1+E05", "1+E06" and "1+E07" represent "1 x 10⁵ PFU", "1 x 10⁶ PFU" and "1 x 10⁷ PFU", respectively.
Figure 8 is a diagram showing the change in the weight of BALB/c mice due to challenge infection with H1N1(2009) pdm, where the mice have been subjected to single vaccination with the recombinant vaccinia virus having H1N1(2009) pdm-derived HA protein gene as a vaccine.
Figure 9 shows pictures of histopathological analysis of lungs of BALB/c mice 9 days after the challenge infection with H1N1(2009) pdm, where the mice have been subjected to single vaccination with the recombinant vaccinia virus having H1N1(2009) pdm-derived HA protein gene as a vaccine. Pneumonia caused by H1N1(2009) pdm infection was significantly reduced in the group inoculated with recombinant vaccinia virus (RVV-Flu HA (H1-mIVR153)).
Figure 10 is a diagram showing the change in the weight of BALB/c mice due to challenge infection with H5N1 HPAIV, where the mice have been subjected to single vaccination with the recombinant vaccinia virus having H5N1 HPAIV-derived HA protein gene as a vaccine.
Figure 11 shows pictures of histopathological analysis of lung of BALB/c mice 9 days after the challenge infection with H5N1 HPAIV, where the mice have been subjected to single vaccination with the recombinant vaccinia virus having H5N1 HPAIV-derived HA protein gene as a vaccine,. Pneumonia caused by highly pathogenic avian influenza virus infection that could be deadly was significantly reduced in the group inoculated with the recombinant vaccinia viruses (RVV-Flu HA (H5-mC12.2), RVV-Flu HA (H5-mC12.3)).

### EMBODIMENTS FOR CARRYING OUT THE INVENTION

Hereinafter, a recombinant vaccinia virus of the present invention and applications thereof will be described in detail.

### 1. Summary of the invention

Among various vaccines, live vaccines are some of the particularly effective vaccines. In general, development of an attenuated vaccine for an emerging virus is known to require a quite long time, which is considered to the same for novel influenzas.

One of known techniques employed in such a case is a genetic engineering technique for preparing a recombinant vaccinia virus (RVV) as a live vaccine. For example, the recombinant vaccinia viruses for rabies virus or rinderpest developed by the present inventors have been demonstrated to exert excellent effect in preventing onset of symptoms caused by infection in field tests and the like (see, for example, Tsukiyama K. et al., Arch. Virol., 1989, vol. 107, p. 225-235).

Moreover, the present inventors has succeeded in preparing a recombinant vaccinia virus having cDNA of SARS-CoV known as a pathogen of atypical pneumonia SARS (WO 2006/038742), and confirmed that it is a formulation that has an excellent prevention effect and that can be used for repeated administration (see, for example, Kitabatake M. et al., Vaccine, 2007, vol. 25, p. 630-637).

A vaccinia virus that is used as a recombinant parent for RVV preparation needs to be a vaccine strain whose safety is ensured. Vaccinia virus LC16m8 strain is known as such a vaccine strain (see, for example, Clinical Virology, vol. 3, No. 3, p. 269, 1975). LC16m8 strain that is isolated from Lister strain is the only vaccine strain currently produced and has actually been administered as a prophylactic vaccine and which has confirmed of its safety and effectiveness. The present inventors also found, in the course of developing and studying a recombinant vaccinia virus for rinderpest, HIV, SARS-CoV and the like that use of gene expression promoter that can greatly enhance antibody-producing capacity and capacity of inducing cell-mediated immunity is beneficial for a vaccinia virus of the present invention. Specifically, they found that pSFJ1-10 or pSFJ2-16 can preferably be used as a plasmid vector (see, for example, Jin N-Y et al., Arch. Virol., 1994, vol. 138, p. 315-330; Elmowalid GA. et al., Pros. Natl. Acad. Sci., 2007, vol. 104, p. 8427-8432; Arch. Virol., vol. 138, p. 315-330, 1994; Japanese Laid-Open Patent Application No. 6-237773).

As a result, the present inventors succeeded in preparing a recombinant vaccinia virus that expresses HA protein derived from H5N1 HPAIV or H1N1(2009) pdm by integrating, into a vaccinia virus, a gene coding for hemagglutinin (HA) protein of highly pathogenic H5N1 avian influenza virus (H5N1 HPAIV) or pandemic H1N1 influenza virus (H1N1(2009) pdm) together with an expression promoter.

A virus that serves as a parent of a recombinant vaccinia virus of the present invention is vaccinia virus as described above. cDNA (entire or a part thereof) coding for HA protein of H5N1 HPAIV or H1N1(2009) pdm may be integrated into the genome of a vaccinia virus so as to give a recombinant vaccinia virus. Each of the cDNAs (the entire or a part thereof) coding for HA protein of H5N1 HPAIV or H1N1(2009) pdm was used as an expression unit and introduced into a vaccinia virus vector. This expression unit was introduced into an HA gene region of vaccinia virus LC16m8 strain. Since introduction of a foreign gene into the HA gene region has no influence on the proliferative activity of the vaccinia virus, use of a safe vaccine strain with weak proliferative capacity as a parent virus of a recombinant virus is already known (see Vaccine, vol. 12, p. 675-681, 1994).

Live recombinant vaccinia vaccines for rabies virus and rinderpest virus have been field-tested and proved of their excellent effect in preventing onset of symptoms caused by infection.

The present inventors inserted HA protein gene of H5N1 HPAIV or H1N1(2009) pdm downstream from the hybrid promoter so as to integrate these genes into the HA protein gene region of the attenuated vaccinia virus strains LC16m8, thereby preparing recombinant vaccinia viruses (RVV). The hybrid promoter includes a poxvirus A-type inclusion (ATI) promoter and tandemly repeated vaccinia virus 7.5 kDa protein (p7.5) early expression promoter (see Jin N-Y et al., Arch. Virol., 1994, vol. 138, p. 315-330). This promoter was developed by and available from Dr. Hisatoshi Shida at Hokkaido University.

When the prepared RVV derived from LC16m8 strain was used to infect animal cells, abundant expression of HA protein of H5N1 HPAIV or H1N1 (2009) pdm was confirmed, while when the prepared RVV was used to vaccinate an individual animal, early production of antibodies with high titer against HA protein of H5N1 HPAIV or H1N1(2009) pdm was confirmed.

### 2. Preparation of recombinant vaccinia virus having HA protein gene of novel influenza virus

HA protein genes of H5N1 HPAIV and H1N1(2009) pdm are already registered with GenBank database (http://www.ncbi.nlm.nih.gov/genbank/) provided by the National Center for Biotechnology Information (NCBI) under designated Accession Numbers. For example, a gene coding for HA protein derived from clade 1 of H5N1 HPAIV (virus strain belonging to clade 1 of subtype H5) (cDNA: SEQ ID NO:1) has been registered under GenBank Accession No. EF541402, a gene coding for HA protein derived from clade 2.1 of H5N1 HPAIV (virus strain belonging to clade 2.1 of subtype H5) (cDNA: SEQ ID NO:2) has been registered under GenBank Accession No. EF541394, and a gene coding for HA protein derived from clade 2.3 of H5N1 HPAIV (virus strain belonging to clade 2.3 of subtype H5) (cDNA: SEQ ID NO:4) has been registered under GenBank Accession No. DQ371928. In addition, a gene coding for HA protein derived from H1N1(2009) pdm (virus strain belonging to subtype H1) (cDNA: SEQ ID NO:5) has been registered under GenBank Accession No. FJ969540.

Genes (cDNAs) coding for HA protein contained in a recombinant vaccinia virus may be the above-mentioned genes as well as a part or a variant sequence thereof. For example, a gene (cDNA) coding for HA protein of clade 1 of H5N1 HPAIV may be DNA having a part of the nucleotide sequence represented by SEQ ID NO:1 deleted (specifically, DNA having the nucleotide sequence represented by SEQ ID NO:12); a gene (cDNA) coding for HA protein of clade 2.1 of H5N1 HPAIV may be DNA having a part of the nucleotide sequence represented by SEQ ID NO:2 deleted (specifically, DNA having the nucleotide sequence represented by SEQ ID NO: 13), and a gene (cDNA) coding for HA protein of clade 2.3 of H5N1 HPAIV may be DNA having a part of the nucleotide sequence represented by SEQ ID NO:4 deleted (specifically, DNA having the nucleotide sequence represented by SEQ ID NO:14). Furthermore, a gene (cDNA) coding for HA protein of H1N1(2009) pdm may be DNA having a part of the nucleotides of DNA having the nucleotide sequence represented by SEQ ID NO:5 mutated (substitutional mutation) (specifically, DNA having the nucleotide sequence represented by SEQ ID NO:15). As a gene coding for HA protein derived from clade 2.2 of H5N1 HPAIV (virus strain belonging to clade 2.2 of subtype H5), a gene having a part of the gene (cDNA) deleted (cDNA: SEQ ID NO:3) has been registered under GenBank Accession No. DQ659327.

These HA protein genes (including partially deleted/mutated genes) have already been cloned and inserted into plasmids. Therefore, a gene contained in a recombinant vaccinia virus, namely, the entire or a part of cDNA coding for HA protein of H5N1 HPAIV or H1N1(2009) pdm (including variant sequences) can be obtained by a general genetic engineering procedure. For example, a nucleic acid synthesis method using a DNA synthesizer, a generally employed genetic engineering procedure, can be employed. Alternatively, a PCR technique in which a gene sequence is isolated or synthesized as a template, primers specific to each gene are designed and the gene sequence is amplified using a PCR device, or a gene amplification method using a cloning vector can be employed. The above-described methods can be carried out with reference to "Molecular cloning, A Laboratory Manual 2nd ed." (Cold Spring Harbor Laboratory Press (1989)) and the like. The resulting PCR product may be purified by a known method. Variant sequences, in particular, substitutionally mutated DNAs, may be prepared, for example, according to site-directed mutagenesis described in "Molecular cloning, A Laboratory Manual 2nd ed." mentioned above, "Current Protocols in Molecular Biology, John Wiley & Sons (1987-1997)", or the like. Specifically, preparation can be carried out by using a mutagenesis kit that makes use of site-directed mutagenesis according to a known technique such as Kunkel method or Gapped duplex method. Preferable examples of such kit include QuickChange™ Site-Directed Mutagenesis Kit (from Stratagene), GeneTailor™ Site-Directed Mutagenesis System (from Invitrogen) and TaKaRa Site-Directed Mutagenesis System (Mutan-K, Mutan-Super Express Km, etc.: from Takara Bio).

HA protein gene of H5N1 HPAIV or HA protein gene of H1N1(2009) pdm inserted into the above-described plasmid can be used as a template for the above-described PCR. Then, PCR is performed using cDNA of HA protein gene of H5N1 HPAIV or H1N1(2009) pdm as a template and primers specific to each gene so as to prepare an HA protein gene region of H5N1 HPAIV or H1N1(2009) pdm. A gene coded by DNA having the nucleotide sequence represented by SEQ ID NO:12 is referred to as "mCl 1" as a gene coding for HA protein derived from clade 1 of H5N1 HPAIV (virus strain belonging to clade 1 of subtype H5), a gene coded by DNA having the nucleotide sequence represented by SEQ ID NO:13 is referred to as "mCl 2.1" as a gene coding for HA protein derived from clade 2.1 of H5N1 HPAIV (virus strain belonging to clade 2.1 of subtype H5), a gene coded by DNA having the nucleotide sequence represented by SEQ ID NO:3 is referred to as "mCl 2.2" as a gene coding for HA protein derived from clade 2.2 of H5N1 HPAIV (virus strain belonging to clade 2.2 of subtype H5), a gene coded by DNA having the nucleotide sequence represented by SEQ ID NO:14 is referred to as "mCl 2.3" as a gene coding for HA protein derived from clade 2.3 of H5N1 HPAIV (virus strain belonging to clade 2.3 of subtype H5), and a gene coded by DNA having the nucleotide sequence represented by SEQ ID NO:15 is referred to as "mIVR153" as a gene coding for HA protein derived from H1N1(2009) pdm (virus strain belonging to subtype H1).

Other than DNAs having the nucleotide sequences represented by SEQ ID NOS:1-5 and 12-15, following DNAs can also be used as DNAs of the above-mentioned HA protein genes (e.g., mCl 1, mCl 2.1, mCl 2.2, mCl 2.3 and mIVR153):
DNA (mutant DNA) that hybridizes to DNA having a nucleotide sequence complementary to the nucleotide sequence represented by SEQ ID NO:1 under stringent conditions and that codes for HA protein derived from clade 1 of H5N1 HPAIV;
DNA (mutant DNA of mCl 1) that hybridizes to DNA having a nucleotide sequence complementary to the nucleotide sequence represented by SEQ ID NO:12 under stringent conditions and that codes for HA protein derived from clade 1 of H5N1 HPAIV;
DNA (mutant DNA) that hybridizes to DNA having a nucleotide sequence complementary to the nucleotide sequence represented by SEQ ID NO:2 under stringent conditions and that codes for HA protein derived from clade 2.1 of H5N1 HPAIV;
DNA (mutant DNA of mCl 2.1) that hybridizes to DNA having a nucleotide sequence complementary to the nucleotide sequence represented by SEQ ID NO: 13 under stringent conditions and that codes for HA protein derived from clade 2.1 of H5N1 HPAIV;
DNA (mutant DNA of mCl 2.2) that hybridizes to DNA having a nucleotide sequence complementary to the nucleotide sequence represented by SEQ ID NO:3 under stringent conditions and that codes for HA protein derived from clade 2.2 of H5N1 HPAIV;
DNA (mutant DNA) that hybridizes to DNA having a nucleotide sequence complementary to the nucleotide sequence represented by SEQ ID NO:4 under stringent conditions and that codes for HA protein derived from clade 2.3 of H5N1 HPAIV;
DNA (mutant DNA of mCl 2.3) that hybridizes to DNA having a nucleotide sequence complementary to the nucleotide sequence represented by SEQ ID NO:14 under stringent conditions and that codes for HA protein derived from clade 2.3 of H5N1 HPAIV;
DNA (mutant DNA) that hybridizes to DNA having a nucleotide sequence complementary to the nucleotide sequence represented by SEQ ID NO:5 under stringent conditions and that codes for HA protein derived from H1N1(2009) pdm (mutant DNA); and
DNA (mutant DNA of mIVR153) that hybridizes to DNA having a nucleotide sequence complementary to the nucleotide sequence represented by SEQ ID NO:15 under stringent conditions and that codes for HA protein derived from H1N1(2009) pdm.

Each of the above-described mutant DNAs can be obtained by chemical synthesis or can be obtained from cDNA library or genomic library by a known hybridization technique such as colony hybridization, plaque hybridization or Southern blotting by using DNA having the nucleotide sequence represented by SEQ ID NO:1-5 or 12-15 or a fragment thereof as a probe. Stringent conditions in the above-described hybridization include, for example, 0.1 x SSC to 10 x SSC, 0.1% to 1.0% SDS and 20°C to 80°C, more specifically, conditions include prehybridization at 37°C to 56°C for 30 minutes or longer followed by one to three times of washing with 0.1 x SSC, 0.1% SDS at room temperature for 10-20 minutes. For detailed procedure of the hybridization method, see, for example, "Molecular Cloning, A Laboratory Manual 2nd ed." (Cold Spring Harbor Press (1989)) or the like.

Moreover, DNA (mutant DNA) having 50% or more, 60% or more, 70% or more, 80% or more, 90% or more, 95% or more, 98% or more or 99% or more homology with the nucleotide sequence represented by SEQ ID NO:1 and coding for HA protein derived from clade 1 of H5N1 HPAIV, DNA (mutant DNA of mCl1) having 50% or more, 60% or more, 70% or more, 80% or more, 90% or more, 95% or more, 98% or more or 99% or more homology with the nucleotide sequence represented by SEQ ID NO:12 and coding for HA protein derived from clade 1 of H5N1 HPAIV, DNA (mutant DNA) having 50% or more, 60% or more, 70% or more, 80% or more, 90% or more, 95% or more, 98% or more or 99% or more homology with the nucleotide sequence represented by SEQ ID NO:2 and coding for HA protein derived from clade 2.1 of H5N1 HPAIV, DNA (mutant DNA of mCl2.1) having 50% or more, 60% or more, 70% or more, 80% or more, 90% or more, 95% or more, 98% or more or 99% or more homology with the nucleotide sequence represented by SEQ ID NO:13 and coding for HA protein derived from clade 2.1 of H5N1 HPAIV, DNA (mutant DNA of mC12.2) having 50% or more, 60% or more, 70% or more, 80% or more, 90% or more, 95% or more, 98% or more or 99% or more homology with the nucleotide sequence represented by SEQ ID NO:3 and coding for HA protein derived from clade 2.2 of H5N1 HPAIV, DNA (mutant DNA) having 50% or more, 60% or more, 70% or more, 80% or more, 90% or more, 95% or more, 98% or more or 99% or more homology with the nucleotide sequence represented by SEQ ID NO:4 and coding for HA protein derived from clade 2.3 of H5N1 HPAIV, DNA (mutant DNA of mC12.3) having 50% or more, 60% or more, 70% or more, 80% or more, 90% or more, 95% or more, 98% or more or 99% or more homology with the nucleotide sequence represented by SEQ ID NO:14 and coding for HA protein derived from clade 2.3 of H5N1 HPAIV, DNA (mutant DNA) having 50% or more, 60% or more, 70% or more, 80% or more, 90% or more, 95% or more, 98% or more or 99% or more homology with the nucleotide sequence represented by SEQ ID NO:5 and coding for HA protein derived from H1N1(2009) pdm, DNA (mutant DNA of mIVR153) having 50% or more, 60% or more, 70% or more, 80% or more, 90% or more, 95% or more, 98% or more or 99% or more homology with the nucleotide sequence represented by SEQ ID NO:15 and coding for HA protein derived from H1N1(2009) pdm can be used.

The expression promoter contained in the recombinant vaccinia virus of the present invention is inserted within the hemagglutinin (HA) gene region of the vaccinia virus and is a hybrid promoter consisting of a poxvirus A-type inclusion (ATI) promoter and a tandemly repeated vaccinia virus 7.5 kDa protein (p7.5) early expression promoter. This promoter can be linked to an appropriate plasmid and, for example, pBMSF7C is known (see Arch. Virol. vol. 138, p. 315-330, 1994; and Japanese Laid-Open Patent Application No. 6-237773).

A nucleotide sequence of a hybrid promoter that can be used with the present invention is represented by SEQ ID NO:6. According to the present invention, however, other than DNA having the nucleotide sequence represented by SEQ ID NO:6, DNA that hybridizes to DNA having a nucleotide sequence complementary to the nucleotide sequence represented by SEQ ID NO:6 and that has a promoter activity may also be used as an expression promoter (particularly, a hybrid promoter). The "stringent conditions" are the same as described above. The phrase "having a promoter activity" means that having an activity of transcribing a gene coding for a structural protein or a non-structural protein.

A protein can be mass-expressed by this hybrid promoter in a completely sugar-modified form from early to late vaccinia virus infection. A plasmid vector having HA protein gene of H5N1 HPAIV or H1N1(2009) pdm inserted downstream from promoter pBMSF7C is called pBMSF7C-mCl1, pBMSF7C-mCl2.1, pBMSF7C-mC12.2, pBMSF7C-mC12.3 or pBMSF7C-mIVR153.

A recombinant vaccinia virus can be prepared by introducing any of these plasmid vectors into a vaccinia virus as a host. Introduction of a plasmid vector into a host can be carried out by employing any known procedure. For example, plasmid vector pBMSF7C-mCl1, pBMSF7C-mCl2.1, pBMSF7C-mC12.2, pBMSF7C-mC12.3 or pBMSF7C-mIVR153 can be introduced into an animal cell infected with attenuated vaccinia virus LC16m8 strain so as to induce homologous recombination in the hemagglutinin (HA) gene region of the vaccinia virus to prepare a recombinant vaccinia virus expressing HA protein gene of H5N1 HPAIV or H1N1(2009) pdm (RVV-Flu HA (H5-mCl1), RVV-Flu HA (H5-mC12.1), RVV-Flu HA (H5-mC12.2), RVV-Flu HA (H5-mC12.3) or RVV-Flu HA (H1-mIVR153)).

Vaccinia virus LC16m8 strain used for preparing RVV can be amplified in an animal individual, but it is an attenuated strain that has extremely low proliferative property in the nerve cells. It is approved as a smallpox vaccine in Japan and no serious side effect has occurred from vaccination of about 50,000 children (see Report from the Research Group for Smallpox, Ministry of Health, Labour and Welfare: Clinical Virology, vol. 3, No. 3, 269, 1975). On the other hand, immune-inducing capacity of LC16m8 strain has been reported to be equivalent to that of the parent strain thereof, i.e., Lister strain, and thus LC16m8 strain is a safe and effective vaccine.

Since HA protein gene of H5N1 HPAIV or H1N1(2009) pdm is inserted into the HA protein gene region of the prepared recombinant vaccinia virus (RVV-Flu HA (H5-mCl1), RVV-Flu HA (H5-mCl2.1), RVV-Flu HA (H5-mC12.2), RVV-Flu HA (H5-mC12.3) or RVV-Flu HA (H1-mIVR153)), expression of the HA protein derived from the vaccinia virus is absent. While influenza virus-derived HA protein causes agglutination of guinea pig erythrocytes, vaccinia virus-derived HA protein does not cause agglutination of guinea pig erythrocytes. Accordingly, RVV is screened by infecting animal cells with RVV-Flu HA (H5-mCl1), RVV-Flu HA (H5-mCl2.1), RVV-Flu HA (H5-mC12.2), RVV-Flu HA (H5-mC12.3) and RVV-Flu HA (H1-mIVR153) so as to use agglutination of guinea pig erythrocytes on the resulting plaque as an indicator. The RVV of interest can be obtained by screening a red plaque that has erythrocyte agglutinating activity.

Introduction of HA protein gene of H5N1 HPAIV or H1N1(2009) pdm in the viruses obtained from the red plaque can be confirmed by performing PCR using a virus genome as a template and primers specific to HA protein gene of H5N1 HPAIV or H1N1(2009) pdm.

Expression of HA protein of H5N1 HPAIV or H1N1(2009) pdm can be confirmed by Western blotting using animal cells infected with RVV-Flu HA (H5-mCl1), RVV-Flu HA (H5-mCl2.1), RVV-Flu HA (H5-mC12.2), RVV-Flu HA (H5-mC12.3) or RVV-Flu HA (H1-mIVR153) as samples. The antibody may be rabbit antiserum prepared by immunizing a HApeptide derived from HA protein of H5N1 HPAIV (a.a. 198-217 (SEQ ID NO:10), etc.) or a HA peptide derived from HA protein of H1N1(2009) pdm (a.a. 223-234 (SEQ ID NO:11), etc.).

Other than HA protein gene region, thymidine kinase (TK) gene region is generally used as the insertion site for gene of interest upon preparation of RVV. However, insertion of gene of interest into the TK gene region is known to cause expression defect of TK, which reduces the proliferative property of RVV. On the other hand, expression defect of HA protein is reported to have little impact on the proliferative property of RVV (see Vaccine, vol. 12, p. 675-681, 1994). Hence, according to the present invention, HA protein gene region is preferable as the insertion site for the gene of interest.

### 3. Pharmaceutical composition for preventing and treating novel influenza

The present invention provides a prophylactic agent and a therapeutic agent (prophylactic and therapeutic pharmaceutical composition) for novel influenza (i.e., highly pathogenic H5N1 avian influenza and pandemic H1N1 influenza), comprising the above-described recombinant vaccinia virus of the invention. A method for preventing and treating the above-mentioned novel influenza comprising a step of administering the above-described recombinant vaccinia virus into a patient (subject), use of the above-described recombinant vaccinia virus for preventing and treating the above-mentioned novel influenza, and use of the above-described recombinant vaccinia virus for producing a prophylactic agent and a therapeutic agent for the above-mentioned novel influenza is also disclosed.

A pharmaceutical composition of the present invention may be introduced into an organism by any known method, for example, by intramuscular, intraperitoneal, intradermal or subcutaneous injection; nasal, buccal or lung inhalation; or oral administration. Additionally, the recombinant vaccinia virus contained in the pharmaceutical composition of the present invention may be used in combination with an existing antiviral drug (e.g., Tamiflu or Relenza). An embodiment of combinational use is not particularly limited, and the recombinant vaccinia virus of the present invention can be administered simultaneously with an existing antiviral drug, or they may be introduced into an organism according to a method in which one is administered after the other after a certain period of time.

Furthermore, a pharmaceutical composition of the present invention may also be blended with a known pharmaceutically acceptable carrier such as an excipient, a bulking agent, a binder or a lubricant, a buffer, a tonicity agent, a chelating agent, a colorant, a preservative, a fragrance, a flavoring agent, a sweetener or the like.

A pharmaceutical composition of the present invention may orally or parenterally be administered according to its form including oral agents such as a tablet, a capsule, a powdered agent, a granular agent, a pill, a solution, syrup or the like, or parenteral agents such as an injection, a topical agent, a suppository or an eye drop. Preferable examples include local injections such as intradermal, intramuscular and intraperitoneal injections.

Although a dosage may appropriately be chosen according to the type of the active element, administration route, subject of administration, age, weight or sex of the patient, symptoms and other conditions, the daily dosage of the virus is about 1000-1000000000 PFU (plaque forming units) and preferably about 100000-100000000 PFU in the case of oral administration, while it is about 100-1000000000 PFU and preferably about 1000-100000000 PFU in the case of parenteral administration. The virus may be administered once or several times a day.

The recombinant vaccinia virus of the present invention is used as a vaccine for preventing or treating novel influenza. So far, development of a vaccine against H5N1 HPAIV or H1N1(2009) involved research focusing on antibodies against H5N1 HPAIV or H1N1(2009) pdm and cytotoxic T cells (CTL). Accordingly, it is preferable that the antibody titer or the cell-mediated immunity activity as a vaccine is measured beforehand.

For example, the antibody titers against the prepared recombinant vaccinia viruses (RVV-Flu HA (H5-mCl1), RVV-Flu HA (H5-mCl2.1), RVV-Flu HA (H5-mCl2.2), RVV-Flu HA (H5-mC12.3) and RVV-Flu HA (H1-mIVR153)) or parent LC16m8 strain can be obtained by immunizing mice with these virus strains, collecting the sera over time, and determining ELISA values against HA protein of H5N1 HPAIV or H1N1(2009) pdm in the sera. In the sera of mice immunized with RVV-Flu HA (H5-mC12.2) and RVV-Flu HA (H5-mC12.3), antibody titers against HA protein of H5N1 HPAIV increased after a week following the immunization, whereas antibody titer against HA protein of H1N1(2009) pdm in the sera of mice immunized with RVV-Flu HA (H1-mIVR153) increased after two weeks following the immunization.

Accordingly, the recombinant vaccinia viruses (collectively referred to as "Flu HA-RVVs") prepared by the present inventors are capable of inducing humoral immune against novel influenza.

Hereinafter, the present invention will be described more specifically by means of examples. These examples are for illustration only and shall not limit the scope of the present invention.

### Example 1: Preparation of recombinant vaccinia virus (RVV)

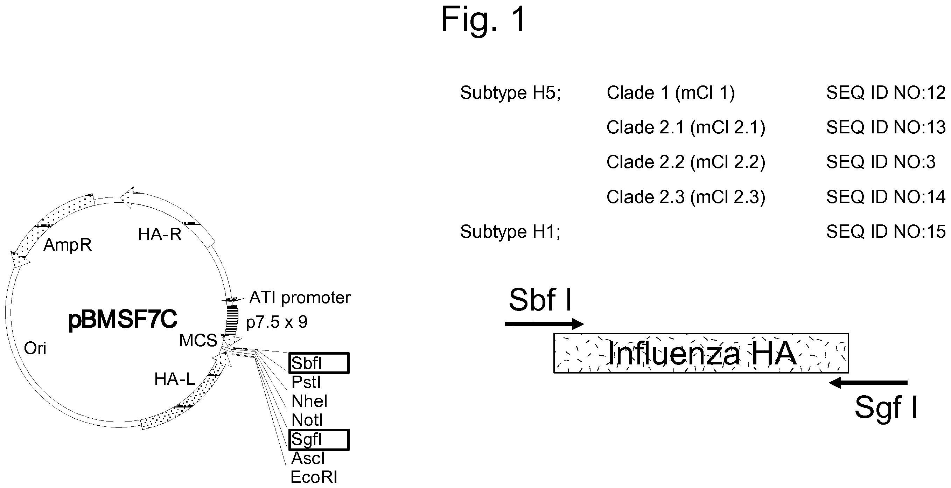

The HA protein gene of either H5N1 HPAIV (clade 1, clade 2.1, clade 2.2, clade 2.3) or H1N1(2009) pdm was integrated into the Sbfl-Sgfl site of pBMSF7C plasmid (see Japanese Laid-Open Patent Application No. 6-237773) so as to prepare plasmid vectors pBMSF7C-mCl1, pBMSF7C-mCl2.1, pBMSF7C-mC12.2, pBMSF7C-mC12.3 and pBMSF7C-mIVR153 in which each of the above-mentioned HA protein genes was inserted downstream from the ATI/p7.5hybrid promoter within the hemagglutinin (HA) gene region (Figure 1). Specifically, DNA having the nucleotide sequence represented by SEQ ID NO:12, as the HA protein gene of clade 1 of H5N1 HPAIV, is inserted into pBMSF7C-mCl1; DNA having the nucleotide sequence represented by SEQ ID NO:13, as the HA protein gene of clade 2.1 of H5N1 HPAIV, is inserted into pBMSF7C-mCl2.1; DNA having the nucleotide sequence represented by SEQ ID NO:3, as the HA protein gene of clade 2.2 of H5N1 HPAIV, is inserted into pBMSF7C-mC12.2; DNA having the nucleotide sequence represented by SEQ ID NO:14, as the HA protein gene of clade 2.3 of H5N1 HPAIV, is inserted into BMSF7C-mC12.3; and DNA having the nucleotide sequence represented by SEQ ID NO:15, as the HA protein gene of H1N1(2009) pdm, is inserted into pBMSF7C-mIVR153.

Primary cultured kidney cells were seeded onto a 35 mm dish. Once the cells reached confluence, the attenuated vaccinia virus LC16m8 strain was infected at moi=10 and 30°C for an hour. Here, moi (multiplicity of infection) refers to PFU (plaque forming units) per cell. Following infection, the virus solution was removed by suction and the cells were washed with PBS(-). Then, after treatment with 5-fold diluted TrypLE/0.5mM EDTA/PBS(-) and washing with 10% FCS/DMEM antibiotic (-) medium, PBS(-) and HeBS buffer, the cells were suspended in 600 µl of HeBS buffer. 1.5 µg each of plasmid vectors pBMSF7C-mCl1, pBMSF7C-mCl2.1, pBMSF7C-mC12.2, pBMSF7C-mC12.3 and pBMSF7C-mIVR153 was diluted with an HeBS buffer to a total amount of 30 µl, which was added to and mixed with the cell suspension and left to stand on ice for 10 minutes. The cell suspension added with the plasmid vector was suctioned with a 10 µl chip-like gold electrode and subjected to electroporation using an electroporator (from Invitrogen) (1200 V; pulse width: 40 ms; and number of pulse: 1). Following electroporation, the resultant was immediately added to RK13 cells or the primary cultured kidney cells that had been seeded onto a 35 mm dish with 2 ml of 10% FCS/DMEM antibiotic(-) medium beforehand. The resultant was added to the 35 mm dish in twice the amount needed for electroporation and cultured at 30°C for 24 hours.

After 24 hours of cultivation, the cells were scraped off from the dish using a scraper and the cell suspension was collected and subjected to ultrasonication (30 sec x 4 times) in cold water followed by centrifugation (2000 rpm, 10 min). The resulting supernatant was used as a virus solution. The virus solution was diluted in 5% FCS/MEM medium and used to infect the primary cultured kidney cells that had been seeded onto a 150 mm dish at 30°C for an hour. The virus solution was removed by suction and then the cells were washed with PBS(-). 5% FCS/0.5% methylcellulose/MEM medium was added and the resultant was cultured at 30°C for 96 hours. After 96 hours of cultivation, the supernatant was removed by suction and washed with PBS(-). A PBS(+)-diluted guinea pig erythrocyte solution was added to the 150 mm dish and cultured at 30°C for 30 minutes. The erythrocyte solution was removed by suction and then the cells were washed twice with PBS(+). Plaques having the guinea pig erythrocytes adsorbed thereon were collected using Pipetman. For the collected plaques, introduction of each of the above-mentioned HA protein genes was confirmed by PCR and gene sequencing. Purification was repeated for three times for plaques in which gene transfer was confirmed.

The viruses subjected to plaque purification for three times were subjected to small-scale cultivation. The colony obtained after the third purification was suspended in 500 µl of 5% FCS/MEM medium and subjected to ultrasonication in cold water. Following centrifugation (2000 rpm, 10 min), 250 µl of the supernatant was added to the primary cultured kidney cells seeded onto a 60 mm dish for infection at 30°C for an hour. After the infection, 2.5 ml of 5% FCS/MEM medium was added and the resultant was cultured at 30°C for 96 hours. 96 hours later, the cells were scraped off from the flask with a scraper to collect the cell suspension. The collected cell suspension was subjected to ultrasonication (30 sec, 4 times) in cold water, followed by centrifugation. The supernatant was collected as a virus solution. The collected virus solution was serially diluted and then added to RK13 cells or the primary cultured kidney cells seeded onto a 6-well plate for infection at 30°C for an hour. The virus solution was removed by suction and the cells were washed twice with PBS(-), to which 5% FCS/0.5% methylcellulose/MEM medium was added for cultivation at 30°C for 96 hours. 96 hours later, the number of plaques formed in the well was counted to calculate the titer.

Based on this calculated titer, mass-scale cultivation was carried out. RK13 cells or the primary cultured kidney cells were seeded onto ten 150 mm dishes. Once the cells reached confluence, the recombinant vaccinia virus solution (2 ml) was used for infection at moi=0.1 and 30°C for an hour. Following infection, the virus solution was removed by suction, added with 18 ml of 5% FCS/MEM medium and cultured at 30°C for 96 hours. 96 hours later, the scraper was used to scrape off the cells from the flask and the cell suspension was collected and stored in a frozen state at -80°C. This cell suspension was subjected to a round of freeze-thaw, ultrasonication (30 sec, 4 times) in cold water and centrifugation. The supernatant was collected as a virus solution. This virus solution was serially diluted and used to infect RK13 cells or the primary cultured kidney cells seeded onto a 6-well plate so as to calculate the titer of the viruses in the solution in the same manner as described above. The virus solutions (recombinant vaccinia viruses) with calculated titers were used in various experiments as described below in the following examples.

### Example 2: Confirmation of introduction of HA protein gene by PCR

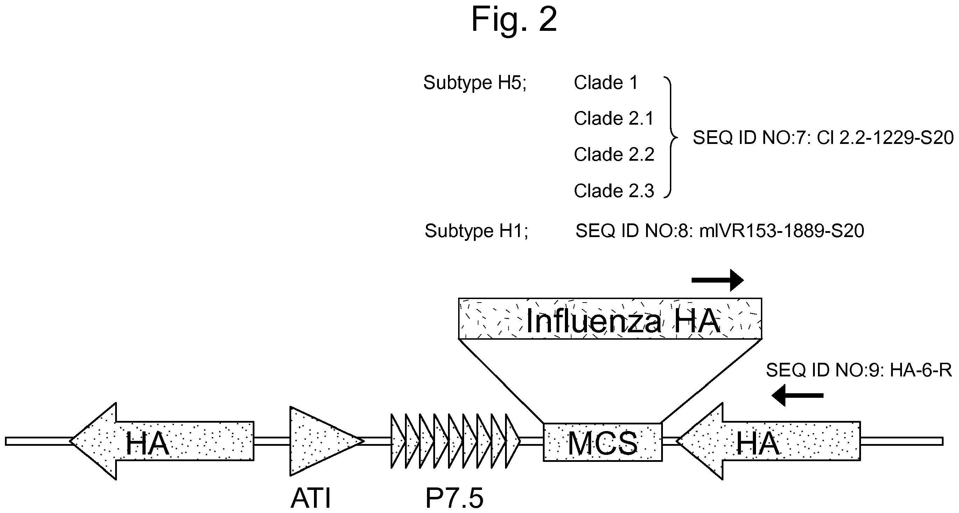

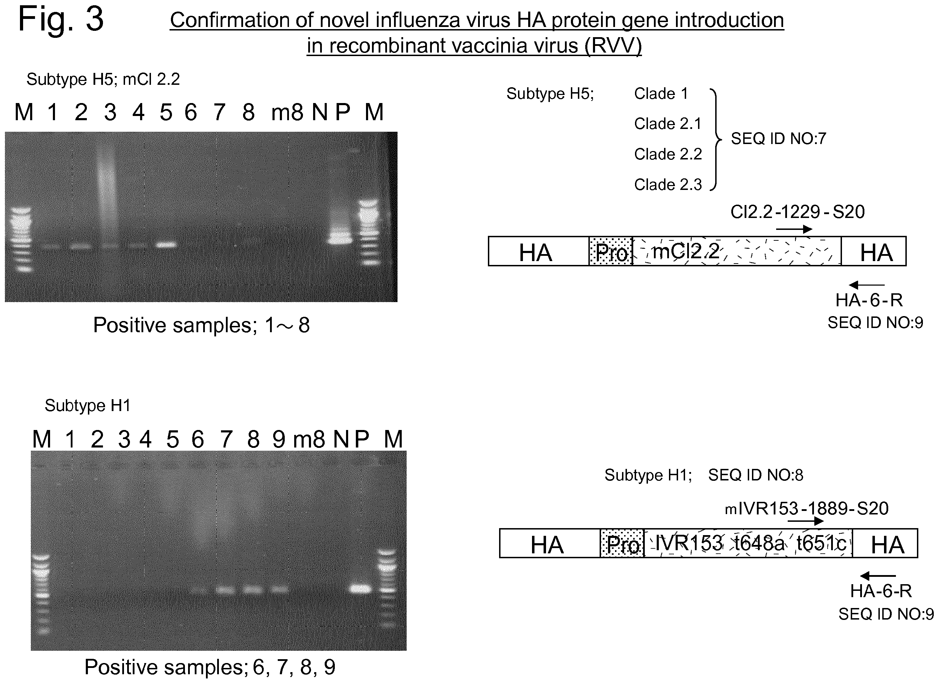

PCR was carried out using the following primers specific to each of the HA protein genes (cDNA: SEQ ID NOS:12, 13, 3, 14 and 15) mentioned in Example 1 and the genome of the resulting recombinant vaccinia virus as a template in order to confirm whether each HA protein gene was introduced into the virus genome (Figures 2 and 3).

### Nucleotide sequences of primers used in PCR for confirming insert

### <F primer>

Fw: C12.2-1229-S20
   5'-CACTCAGTTTGAGGCCGTTG-3' (SEQ ID NO:7)
   (for confirming HA protein genes of H5N1 HPAIV (4 types))
Fw: mIVR153-1889-S20
   5'- AATGCGAACTGTTGGTTCT- 3' (SEQ ID NO:8)
   (for confirming HA protein gene of H1N1(2009) pdm)

### <R primer>

Rv: HA-6-R
5'- CTAGTTCTGAGAAACCAGAGG-3' (SEQ ID NO:9)
(for confirming HA protein genes of H5N1 HPAIV and H1N1(2009) pdm)

Specifically, the composition of the reaction solution was 1 U DNA polymerase, 0.3 mM dNTP, 1 µM F-primer and 1 µM R-primer in 50 µl of a buffer attached to a commercially available polymerase. The cycle conditions were a total of 25 cycles of: denaturing at 95°C for 0.5 minutes; annealing at 58°C for 0.5 minutes; and elongation at 72°C for 2 minutes. The resulting PCR product was subjected to electrophoresis using agarose gel to confirm the band. As a result, HA protein gene of H5N1 HPAIV or H1N1(2009) pdm was found to be introduced into the recombinant virus genome if a single band having the length anticipated by primer design was observed whereas HA protein gene of H5N1 HPAIV or H1N1(2009) pdm was found to be not introduced if no such band was observed.

As shown in Figure 3, as a result of 1% agarose gel electrophoresis of the PCR product (amplified fragment), HA protein gene of H5N1 HPAIV or H1N1(2009) pdm was found to be introduced into the recombinant virus genome.

### Example 3: Confirmation of expression of HA protein of H5N1 HPAIV or H1N1(2009) pdm by Western blotting

The recombinant vaccinia virus was used to infect RK13 cells that had been seeded onto a 6-well plate beforehand at moi=10 and 30°C for an hour. Following infection, the virus solution was removed by suction and the cells were washed once with PBS(-). To each well, 2 ml of 5% FCS/MEM medium was added for cultivation at 30°C for 18 hours. 18 hours later, the medium was removed by suction and the cells were washed twice with PBS(-). 100 µl of lysis buffer (1% SDS, 0.5% NP-40, 0.15 M NaCl, 10 mM Tris-HCl (pH 7.4)) was added to lyse the cells, and the solution was transferred into a 1.5 ml Eppendorf tube. The collected solution was subjected to ultrasonication in cold water until zero viscosity. The amount of protein in the prepared solution was quantified according to Lowry method.

Electrophoresis was carried out for 30 µg of the protein using 10% acrylamide gel. At the end of electrophoresis, the gel was removed, and the protein in the gel was transferred onto a PVDF membrane with a semi-dry blotter by running a current at 2 mA/cm² for 60 minutes. The membrane was washed with a TBS-T solution and immersed in a 5% skimmed milk-TBS-T solution for blocking. At the end of the blocking, the membrane was washed for three times with a TBS-T solution. The primary antibody was a rabbit polyclonal antibody against a HA peptide derived from HA protein of H5N1 HPAIV (a.a. 198-217 (SEQ ID NO:10; NDAAEQTKLYQNPTTYISVG)) or a HA peptide derived from HA protein of H1N1(2009) pdm (a.a. 223-234 (SEQ ID NO:11; YSKKFKPEIAIR)). At the end of the reaction with the primary antibody, the membrane was washed for three times with a TBS-T solution. The secondary antibody was anti-rabbit IgG-linked HRPO (from Donkey, Amersham). At the end of the reaction with the secondary antibody, the membrane was again washed for three times with a TBS-T solution. Immobilon western detection reagent (from Millipore) was added to the membrane and the fluorescence was captured with a CCD camera for image analysis.

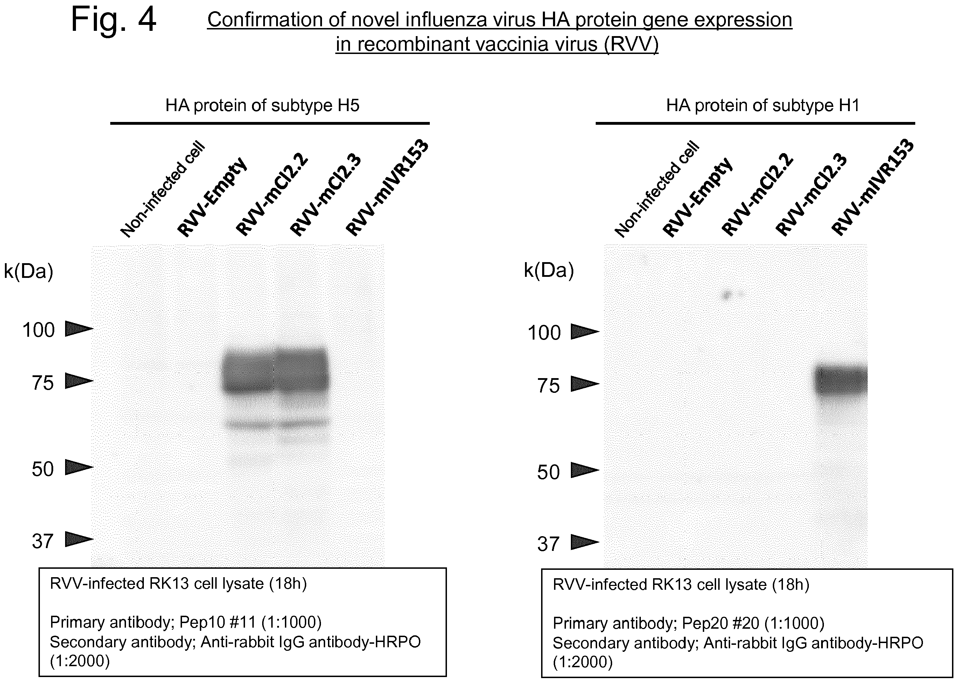

As a result, as shown in Figure 4, HA proteins of H5N1 HPAIV and H1N1(2009) pdm were found to be expressed in the recombinant virus genomes of RVV-Flu HA (H5-mC12.2), RVV-Flu HA (H5-mC12.3) and RVV-Flu HA (H1-mIVR153).

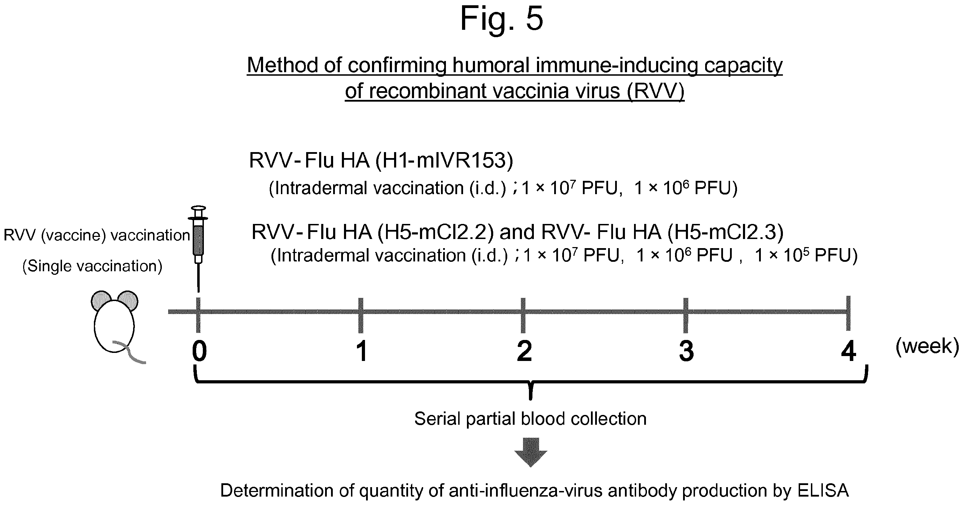

### Example 4: Experiments of vaccination of mice with recombinant vaccinia viruses (Figure 5)

Figure 5 is a diagram showing a method of confirming humoral immune-inducing capacity of recombinant vaccinia viruses (Flu HA-RVVs).

The recombinant vaccinia virus obtained in Example 1 or RVV-Empty in which an empty vector had been inserted was used to immunize BALB/c mice (female) transendothelially (intradermally (i.d.)) at 1 x 10⁷ PFU, and 1, 2, 3 and 4 weeks later, blood was drawn from the orbital vein. The collected blood was all placed into a 0.5 ml tube containing a serum separation agent and subjected to centrifugation (15000 rpm, 10 minutes) to separate and collect the serum. The serum stored in a frozen state at -80°C until the subsequent ELISA test described below.

### Example 5: Determination of antibody titer against HA protein of H5N1 HPAIV or H1N1(2009) pdm in sera of mice immunized with recombinant vaccinia viruses (Flu HA-RVVs) by ELISA method

A 96-well plate was coated with HA protein of H5N1 HPAIV or H1N1(2009) pdm, to which 100-1000-fold dilution of the frozen serum sample was added and left to stand at 4°C overnight. The 96-well plate was washed with a TBS-T solution, and then added with anti-mouse IgG-linked HRPO (from Sheep, Amersham) as a secondary antibody. Following reaction at room temperature for 2 hours, the 96-well plate was again washed for three times with a TBS-T solution. A color development solution was added at 100 µl/well. After leaving it to stand at room temperature for 30 minutes, 2M sulfuric solution was added at 50 µl/well to terminate the reaction. Absorbance at 490 nm was read with a microplate reader.

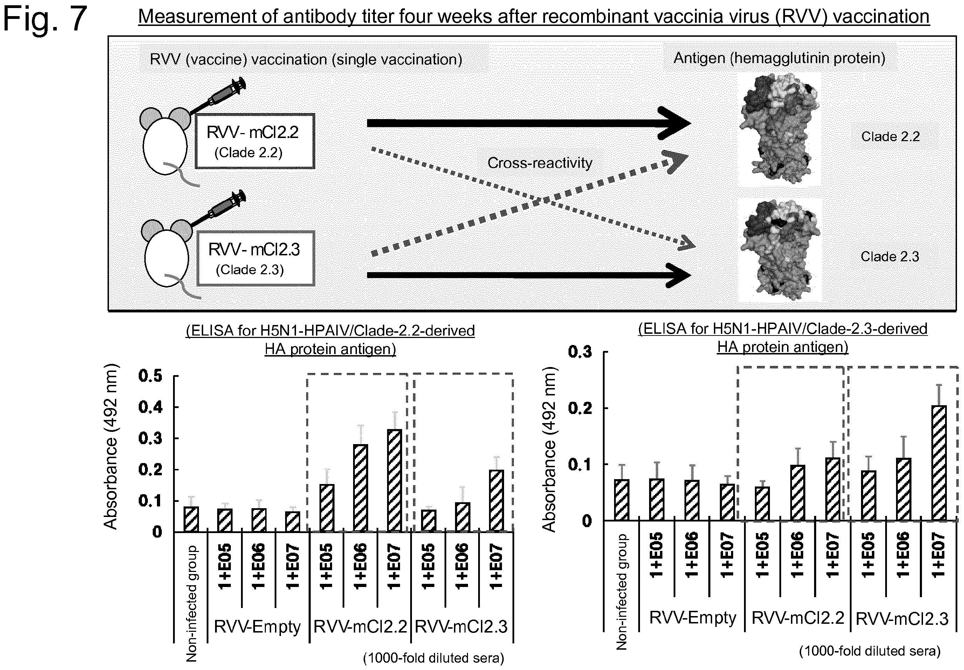

As a result, as shown in Figures 6 and 7, antibody titers were highly induced against HA protein of H5N1 HPAIV or H1N1(2009) pdm in the mice inoculated with RVV-Flu HA (H5-mC12.2), RVV-Flu HA (H5-mC12.3) and RVV-Flu HA (H1-mIVR153).

### Example 6: Examination of prophylactic effect of recombinant vaccinia viruses (RVV-Flu HA (H5-mCl2.2), RVV-Flu HA (H5-mCl2.3) and RVV-Flu HA (H1-mIVR153)) against novel influenza

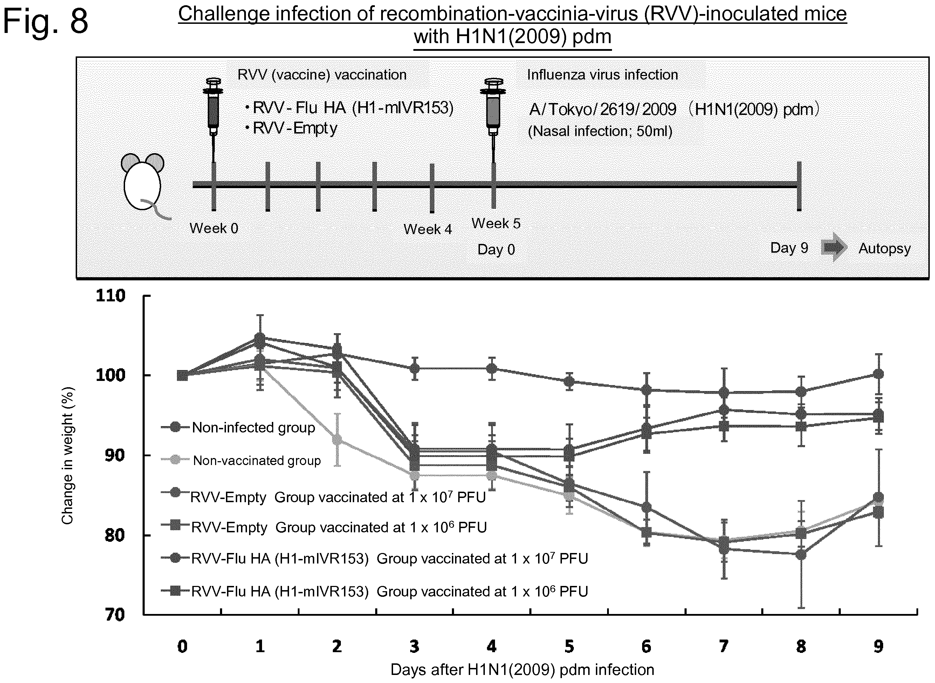

As shown in Figure 8, since RVV-Flu HA (H1-mIVR153) vaccination was confirmed to have an effect in immune induction, mice inoculated with these recombinant vaccinia viruses (vaccines) were subjected to challenge infection through the noses with H1N1(2009) pdm following 5 weeks after the vaccination. Changes in the weights were measured over days, and autopsy was performed 9 days after the vaccination for pathological analysis of the lungs. While the weights of the non-vaccinated mice decreased over days after the influenza virus infection with approximately 20% of decrease on Day 9, the vaccinated group temporarily showed weight loss in the early days after the H1N1(2009) pdm infection but rapidly regained the weight to almost the normal value

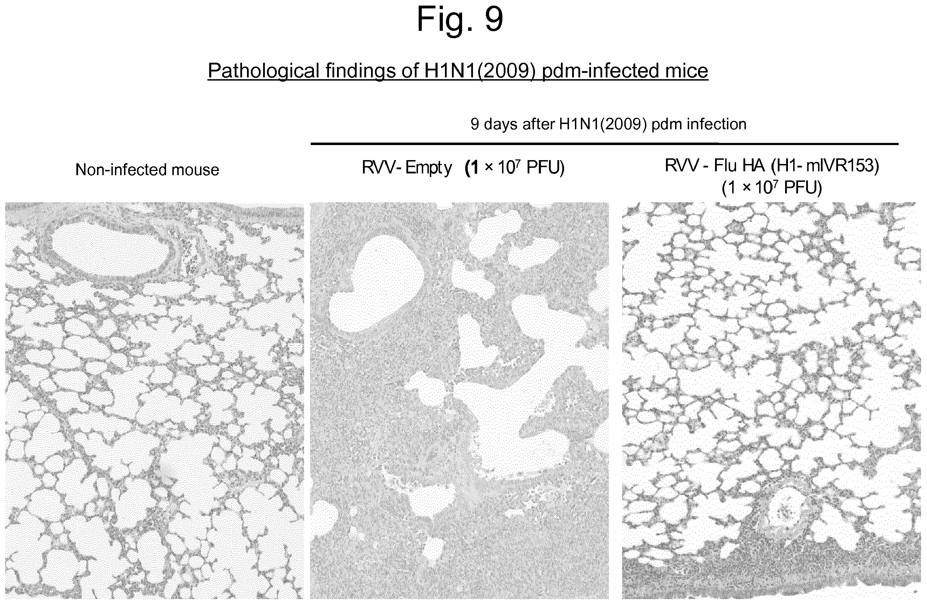

In addition, it was found, from the histopathological analysis of the lungs, that the lungs of the non-vaccinated mice infected with H1N1(2009) pdm exhibited a pneumonia image with thickened stromal cells, mucus exudation and locally filled alveolar space due to infiltration with the lymphocytes, i.e., immunocompetent cells, whereas pneumonia was found to be significantly alleviated in the vaccinated group where significant lymphocytic infiltration was observed around the bronchi, i.e., the infected site, but the alveolar space stayed clear (Figure 9).

Furthermore, mice subjected to single vaccination with RVV-Flu HA (H5-mC12.2) or RVV-Flu HA (H5-mC12.3) were subjected to challenge infection with clade 2.3 of H5N1 HPAIV after five weeks following vaccination with these recombinant vaccinia viruses (vaccines) to examine the effects of the vaccines. The challenge infection was performed at two concentrations, i.e., 1 x 10⁴ PFU and 1 x 10⁵ PFU. In either case of challenge infection at 1 x 10⁴ PFU or 1 x 10⁵ PFU, survival rates were 100% for both of the groups inoculated with RVV-Flu HA (H5-mC12.2) and RVV-Flu HA (H5-mC12.3), and the weights increased after three days following the influenza virus infection and rebounded to almost the same weight before the infection. Additionally, it was found, from the histopathological analysis of the lungs upon challenge infection at the higher concentration, i.e., 1 x 10⁵ PFU, that the lungs of the non-vaccinated mice infected with H5N1 virus showed thickened stromal cells even in the lungs of the survived individuals , whereas pneumonia was significantly alleviated in the groups vaccinated with RVV-Flu HA (H5-mC12.2) or RVV-Flu HA (H5-mC12.3) where significant lymphocytic infiltration around the bronchi, i.e., the infected site, and the vessels and locally thicken stroma were observed but the alveolar space stayed relatively clear. Accordingly, the recombinant vaccinia virus (vaccine) prepared this time also showed protective effect against deadly H5N1 HPAIV infection. A sufficient vaccine effect against infection with clade 2.3 virus was also observed in the group inoculated with different clade, i.e., clade 2.2.

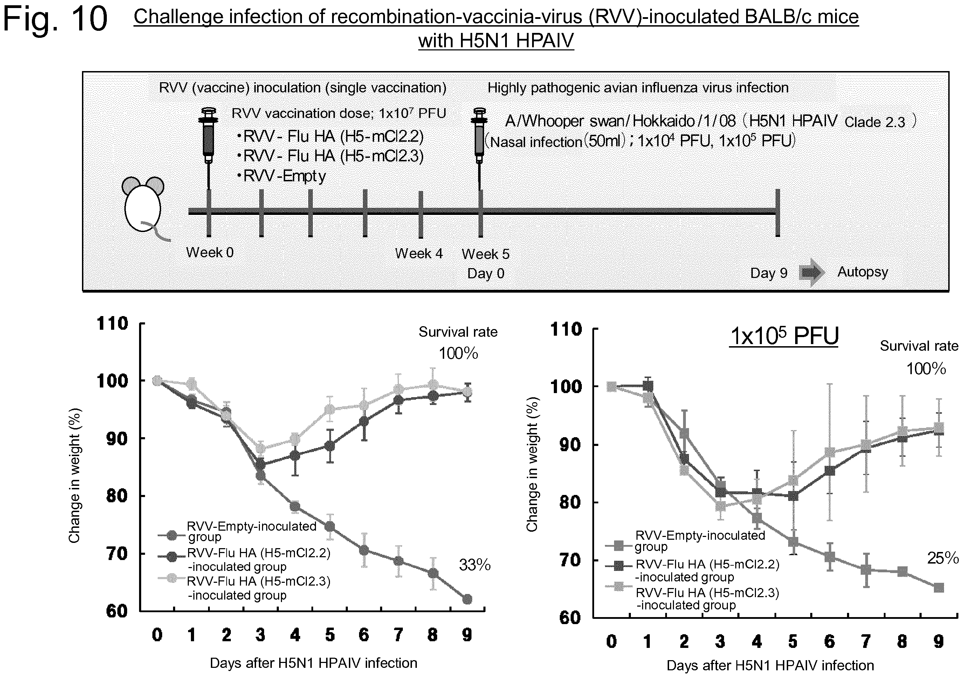

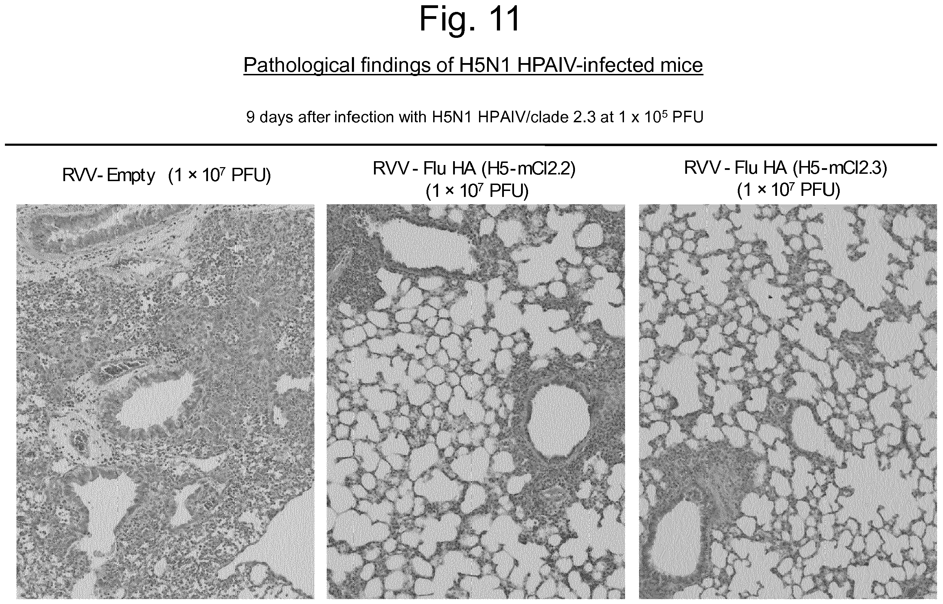

As the novel influenza recombinant vaccinia viruses, RVV-Flu HA (H5-mC12.2), RVV-Flu HA (H5-mC12.3) and RVV-Flu HA (H1-mIVR153) that express hemagglutinin (HA) protein were used (Figure 1). In order to evaluate the effects of these Flu HA-RVVs, RVV-Flu HA (H5-mC12.2), RVV-Flu HA (H5-mC12.3) or RVV-Flu HA (H1-mIVR153) was subcutaneously used for single vaccination at 1 x 10⁷ PFU. After five weeks following the vaccination, H5N1 HPAIV or H1N1(2009) pdm was used for infection through the noses, and then the changes in the weights were measured over days, and the lung tissues were collected 9 days after the infection (Figures 8-11) for histopathological analysis of the lungs (Figures 9 and 11).

With respect to the challenge infection with each influenza virus, reduction in weight loss and an effect in alleviating pneumonia were observed in all of the RVV-Flu HA (H5-mC12.2)-, RVV-Flu HA (H5-mC12.3)- and RVV-Flu HA (H1-mIVR153)-inoculated groups (Figures 8-11).

Thus, a vaccinia virus of the present invention was demonstrated to have prophylactic and therapeutic effects against novel influenza and shown to be useful as a novel influenza vaccine.

### INDUSTRIAL APPLICABILITY

The present invention provides a highly-safe novel recombinant vaccinia virus that is effective in preventing or treating novel influenza, and a prophylactic agent or a therapeutic agent for novel influenza comprising the same (a prophylactic or therapeutic vaccine against novel influenza).

### SEQUENCE LISTING

SEQ ID NO:7: Synthetic DNA
SEQ ID NO:8: Synthetic DNA
SEQ ID NO:9: Synthetic DNA
SEQ ID NO:12: Recombinant DNA
SEQ ID NO:13: Recombinant DNA
SEQ ID NO:14: Recombinant DNA
SEQ ID NO:15: Recombinant DNA

## Claims

1. A recombinant vaccinia virus LC16m8 strain, comprising (i) DNA having the nucleotide sequence of SEQ ID NO:3 and coding for HA protein derived from clade 2.2 of H5N1 HPAIV; or (ii) DNA having the nucleotide sequence of SEQ ID NO:14 and coding for HA protein derived from clade 2.3 of H5N1 HPAIV.

2. The recombinant vaccinia virus according to claim 1, wherein the recombinant vaccinia virus comprises an expression promoter which is a hybrid promoter.

3. The recombinant vaccinia virus according to claim 2, wherein the hybrid promoter is one having either DNA (a) or (b) below:
(a) DNA having the nucleotide sequence of SEQ ID NO:6; or
(b) DNA that hybridizes to DNA having a nucleotide sequence complementary to the nucleotide sequence of SEQ ID NO:6 under stringent conditions and that has a promoter activity.

4. The recombinant vaccinia virus according to any one of the preceding claims, for use in a method of preventing highly pathogenic H5N1 avian influenza.

5. The recombinant vaccinia virus according to any one of claims 1 to 3, for use in a method of treating highly pathogenic H5N1 avian influenza.

6. A pharmaceutical composition comprising a recombinant vaccinia virus as defined in any one of claims 1 to 4.

7. The pharmaceutical composition according to claim 6, for use in a method of preventing highly pathogenic H5N1 avian influenza.

8. The pharmaceutical composition according to claim 6, for use in a method of treating highly pathogenic H5N1 avian influenza.

## Patentansprüche

1. Rekombinantes Vacciniavirus-LC16m8-Stamm, umfassend (i) DNA mit der Nukleotidsequenz von SEQ ID NO:3 und kodierend für HA-Protein, abgeleitet von Klade 2.2 von H5N1 HPAIV; oder (ii) DNA mit der Nukleotidsequenz von SEQ ID NO:14 und kodierend für HA-Protein, abgeleitet von Klade 2.3 von H5N1 HPAIV.

2. Rekombinantes Vacciniavirus nach Anspruch 1, wobei das rekombinante Vacciniavirus einen Expressionspromotor umfasst, der ein Hybridpromotor ist.

3. Rekombinantes Vacciniavirus nach Anspruch 2, wobei der Hybridpromotor einer ist, der entweder die nachstehende DNA (a) oder (b) aufweist:
(a) DNA mit der Nukleotidsequenz von SEQ ID NO: 6; oder
(b) DNA, die mit DNA mit einer Nukleotidsequenz hybridisiert, die zu der Nukleotidsequenz von SEQ ID NO:6 unter stringenten Bedingungen komplementär ist, und eine Promotoraktivität aufweist.

4. Rekombinantes Vacciniavirus nach einem der vorhergehenden Ansprüche zur Verwendung in einem Verfahren zur Verhinderung von hoch pathogener H5N1-Vogelgrippe.

5. Rekombinantes Vacciniavirus nach einem der Ansprüche 1 bis 3 zur Verwendung in einem Verfahren zur Behandlung von hoch pathogener H5N1-Vogelgrippe.

6. Pharmazeutische Zusammensetzung umfassend ein rekombinantes Vacciniavirus nach einem der Ansprüche 1 bis 4.

7. Pharmazeutische Zusammensetzung nach Anspruch 6 zur Verwendung in einem Verfahren zur Verhinderung von hoch pathogener H5N1-Vogelgrippe.

8. Pharmazeutische Zusammensetzung nach Anspruch 6 zur Verwendung in einem Verfahren zur Behandlung von hoch pathogener H5N1-Vogelgrippe.

## Revendications

1. Souche LC16m8 du virus de la vaccine recombinant, comprenant (i) un ADN ayant la séquence nucléotidique de SEQ ID N° : 3 et codant pour une protéine HA dérivée du clade 2.2 de H5N1 HPAIV ; ou (ii) de l'ADN ayant la séquence nucléotidique de SEQ ID N° : 14 et codant pour une protéine HA dérivée du clade 2.3 de H5N1 HPAIV.

2. Virus de la vaccine recombinant selon la revendication 1, le virus de vaccine recombinant comprenant un promoteur d'expression qui est un promoteur hybride.

3. Virus de la vaccine recombinant selon la revendication 2, dans lequel le promoteur hybride est un promoteur ayant un ADN (a) ou (b) ci-dessous :
(a) un ADN ayant la séquence nucléotidique de SEQ ID N° : 6 ; ou
(b) un ADN qui s'hybride à l'ADN ayant une séquence nucléotidique complémentaire de la séquence nucléotidique de SEQ ID N° : 6 dans des conditions strictes et qui présente une activité de promoteur.

4. Virus de la vaccine recombinant selon l'une quelconque des revendications précédentes, destiné à une utilisation dans un procédé de prévention d'une influenza aviaire H5N1 hautement pathogène.

5. Virus de la vaccine recombinant selon l'une quelconque des revendications 1 à 3, destiné à une utilisation dans un procédé de traitement d'une influenza aviaire H5N1 hautement pathogène.

6. Composition pharmaceutique comprenant un virus de la vaccine recombinant tel que défini dans l'une quelconque des revendications 1 à 4.

7. Composition pharmaceutique selon la revendication 6, destinée à une utilisation dans un procédé de prévention d'une influenza aviaire H5N1 hautement pathogène.

8. Composition pharmaceutique selon la revendication 6, destinée à une utilisation dans un procédé de traitement d'une influenza aviaire H5N1 hautement pathogène.


### SEQUENCE LISTING

<110> Tokyo Metropolitan Institute of Medical Science The Chemo- Sero- Therapeutic Research Institute National University Corporation Hokkaido University
<120> A recombinant vaccinia virus having a hemagglutinin gene derived from a new influenza virus
<130> PCT11-0055
<150> JP 2010-233064
   <151> 2010-10-15
<160> 15
<170> PatentIn version 3.4
<210> 1
   <211> 1707
   <212> DNA
   <213> Influenza A virus
<400> 1
   atggagaaaa tagtgcttct ttttgcaata gtcagtcttg ttaaaagtga tcagatttgc    60
   attggttacc atgcaaacaa ctcgacagag caggttgaca caataatgga aaagaacgtt   120
   actgttacac atgcccaaga catactggaa aagacacaca atgggaagct ctgcgatcta   180
   gatggagtga agcctctaat tttgagagat tgtagtgtag ctggatggct cctcggaaac   240
   ccaatgtgtg acgaattcat caatgtgccg gaatggtctt acatagtgga gaaggccaat   300
   ccagtcaatg acctctgtta cccaggggat ttcaatgact atgaagaatt gaaacaccta   360
   ttgagcagaa taaaccattt tgagaaaatt cagatcatcc ccaaaagttc ttggtccagt   420
   catgaagcct cattgggggt gagctcagca tgtccatacc agggaaagtc ctcctttttc   480
   agaaatgtgg tatggcttat caaaaagaac agtacatacc caacaataaa gaggagctac   540
   aataatacca accaagaaga tcttttggta ctgtggggga ttcaccatcc taatgatgcg   600
   gcagagcaga caaagctcta tcaaaaccca accacctata tttccgttgg gacatctaca   660
   ctaaaccaga gattggtacc aagaatagct actagatcca aagtaaacgg gcaaagtgga   720
   aggatggagt tcttctggac aattttaaaa ccgaatgatg caatcaactt cgagagtaat   780
   ggaaatttca ttgctccaga atatgcatac aaaattgtca agaaagggga ctcaacaatt   840
   atgaaaagtg aattggaata tggtaactgc aacaccaagt gtcaaactcc aatgggggcg 900
   ataaactcta gcatgccatt ccacaatata caccctctca ccatcgggga atgccccaaa 960
   tatgtgaaat caaacagatt agtccttgcg actgggctca gaaatagccc tcaaagagag 1020
   agaagaagaa aaaagagagg attatttgga gctatagcag gttttataga gggaggatgg 1080
   cagggaatgg tagatggttg gtatgggtac caccatagca acgagcaggg gagtgggtac 1140
   gctgcagaca aagaatccac tcaaaaggca atagatggag tcaccaataa ggtcaactcg 1200
   attattgaca aaatgaacac tcagtttgag gccgttggaa gggaatttaa caacttagaa 1260
   aggagaatag agaatttaaa caagaagatg gaagacgggt tcctagatgt ctggacttat 1320
   aatgctgaac ttctagttct catggaaaac gagagaactc tagactttca tgactcaaat 1380
   gtcaagaacc tttacgacaa ggtccgacta cagcttaggg ataatgcaaa ggagctgggt 1440
   aacggttgtt tcgagttcta tcataaatgt gataatgaat gtatggaaag tgtaagaaac 1500
   ggaacgtatg actacccgca gtattcagaa gaagcaagac taaaaagaga ggaaataagt 1560
   ggagtaaaat tggaatcaat aggaatttac caaatattgt caatttattc tacagtggcg 1620
   agctccctag cactggcaat catggtagct ggtctatcct tatggatgtg ctccaatggg 1680
   tcgttacaat gcagaatttg catttaa 1707
<210> 2
   <211> 1707
   <212> DNA
   <213> Influenza A virus
<400> 2
   atggagaaaa tagtgcttct tcttgcaata gtcagtcttg ttaaaagtga tcagatttgc 60
   attggttacc atgcaaacaa ttcaacagag caggttgaca caatcatgga aaagaacgtt 120
   actgttacac atgcccaaga catactggaa aagacacaca acgggaagct ctgcgatcta 180
   gatggagtga agcctctaat tttaagagat tgtagtgtag ctggatggct cctcgggaac 240
   ccaatgtgtg acgaattcat caatgtaccg gaatggtctt acatagtgga gaaggccaat 300
   ccaaccaatg acctctgtta cccagggagt ttcaacgact atgaagaact gaaacaccta 360
   ttgagcagaa taaaccattt tgagaaaatt caaatcatcc ccaaaagttc ttggtccgat 420
   catgaagcct catcaggagt gagctcagca tgtccatacc tgggaagtcc ctcctttttt 480
   agaaatgtgg tatggcttat caaaaagaac agtacatacc caacaataaa gaaaagctac 540
   aataatacca accaagaaga tcttttggta ctgtggggaa ttcaccatcc taatgatgcg 600
   gcagagcaga caaggctata tcaaaaccca accacctata tttccattgg gacatcaaca 660
   ctaaaccaga gattggtacc aaaaatagct actagatcca aagtaaacgg gcaaagtgga 720
   aggatggagt tcttctggac aattttaaaa cctaatgatg caatcaactt cgagagtaat 780
   ggaaatttca ttgctccaga atatgcatac aaaattgtca agaaagggga ctcagcaatt 840
   atgaaaagtg aattggaata tggtaactgc aacaccaagt gtcaaactcc aatgggggcg 900
   ataaactcta gtatgccatt ccacaacata caccctctca ccatcgggga atgccccaaa 960
   tatgtgaaat caaacagatt agtccttgca acagggctca gaaatagccc tcaaagagag 1020
   agcagaagaa aaaagagagg actatttgga gctatagcag gttttataga gggaggatgg 1080
   cagggaatgg tagatggttg gtatgggtac caccatagca atgagcaggg gagtgggtac 1140
   gctgcagaca aagaatccac tcaaaaggca atagatggag tcaccaataa ggtcaactca 1200
   atcattgaca aaatgaacac tcagtttgag gccgttggaa gggaatttaa taacttagaa 1260
   aggagaatag agaatttaaa caagaagatg gaagacgggt ttctagatgt ctggacttat 1320
   aatgccgaac ttctggttct catggaaaat gagagaactc tagactttca tgactcaaat 1380
   gttaagaacc tctacgacaa ggtccgacta cagcttaggg ataatgcaaa ggagctgggt 1440
   aacggttgtt tcgagttcta tcacaaatgt gataatgaat gtatggaaag tataagaaac 1500
   ggaacgtaca actatccgca gtattcagaa gaagcaagat taaaaagaga ggaaataagt 1560
   ggggtaaaat tggaatcaat aggaacttac caaatactgt caatttattc aacagtggcg 1620
   agttccctag cactggcaat catgatggct ggtctatctt tatggatgtg ctccaatgga 1680
   tcgttacaat gcagaatttg catttaa 1707
<210> 3
   <211> 1695
   <212> DNA
   <213> Influenza A virus
<400> 3
   atggagaaaa tagtgcttct tcttgcaata gtcagtcttg ttaaaagtga tcagatttgc 60
   attggttacc atgcaaacaa ctcgacagag caggttgaca caataatgga aaagaacgtc 120
   actgttacac acgcccaaga catactggaa aagacacaca acgggaagct ctgcgatcta 180
   gatggagtga agcctctaat tttaagagat tgtagtgtag ctggatggct cctcgggaac 240
   ccaatgtgtg acgaattcct caatgtgccg gaatggtctt acatagtgga gaagatcaat 300
   ccagccaatg acctctgtta cccagggaat ttcaacgact atgaagaact gaaacaccta 360
   ttgagcagaa taaaccattt tgagaaaatt cagatcatcc ccaaaagttc ttggtcagat 420
   catgaagcct catcaggggt gagctcagca tgtccatacc agggaaggtc ctcctttttt 480
   agaaatgtgg tatggcttat caaaaagaac aatgcatacc caacaataaa gagaagttac 540
   aataatacca accaagaaga tcttttggta ctgtggggga ttcaccatcc aaatgatgcg 600
   gcagagcaga caaggctcta tcaaaaccca accacctata tttccgttgg gacatcaaca 660
   ctaaaccaga gattggtacc aaaaatagct actagatcca aggtaaacgg gcaaagtgga 720
   aggatggagt tcttttggac aattttaaaa ccgaatgatg caataaactt tgagagtaat 780
   ggaaatttca ttgctccaga aaatgcatac aaaattgtca agaaagggga ctcaacaatt 840
   atgaaaagtg aattggaata tggtaactgc aacaccaagt gtcaaactcc aataggggcg 900
   ataaactcta gtatgccatt ccacaacatc caccctctca ccatcgggga atgccccaaa 960
   tatgtgaaat caaacagatt agtccttgcg actgggctca gaaatagccc tcaaattgaa 1020
   actagaggat tatttggagc tatagcaggt tttatagagg gaggatggca gggaatggta 1080
   gatggttggt atgggtacca ccatagcaac gagcagggga gtgggtacgc tgcagacaaa 1140
   gaatccactc aaaaggcaat agatggagtc accaataagg tcaactcgat cattgacaaa 1200
   atgaacactc agtttgaggc cgttggaagg gaatttaata acttagaaag gagaatagaa 1260
   aatttaaaca agaagatgga agacggattc ctagatgtct ggacttataa tgctgaactt 1320
   ctggttctca tggaaaatga gagaactcta gactttcatg actcaaatgt caagaacctt 1380
   tacgacaagg tccgactaca gcttagggat aatgcaaagg agcttggtaa cggttgtttc 1440
   gagttctatc acagatgtga taatgaatgt atggaaagtg taagaaacgg aacgtatgac 1500
   tacccgcagt attcagaaga agcaagatta aaaagagagg aaataagtgg agtaaaattg 1560
   gaatcaatag gaacttacca aatactgtca atttattcaa cagtggcgag ctccctagca 1620
   ctggcaatca tggtggctgg tctatctttg tggatgtgct ccaatggatc gttacaatgc 1680
   agaatttgca tttaa 1695
<210> 4
   <211> 1704
   <212> DNA
   <213> Influenza A virus
<400> 4
   atggagaaaa tagtgcttct tcttgcaata gtcagccttg ttaaaagtga tcagatttgc     60
   attggttacc atgcaaacaa ctcgacagag caggttgaca caataatgga aaagaacgtt    120
   actgttacac atgcccaaga catactggaa aagacacaca acgggaagct ctgcgatcta    180
   gatggagtga agcctctgat tttaagagat tgtagtgtag ctggatggct cctcggaaac    240
   ccaatgtgtg acgaattcat caatgtgccg gaatggtctt acatagtgga gaaggccaac    300
   ccagccaatg acctctgtta cccagggaat ttcaacgact atgaagaact gaaacaccta    360
   ttgagcagaa taaaccattt tgagaaaatt cagatcatcc ccaaaagttc ttggtccgat    420
   catgaagcct catcaggggt gagctcagca tgtccatacc agggaacgcc ctcctttttc    480
   agaaatgtgg tatggcttat caaaaagaac aatacatacc caacaataaa gagaagctac    540
   aataatacca accaggaaga tcttttgata ctgtggggga ttcatcattc taatgatgcg    600
   gcagagcaga caaagctcta tcaaaaccca accacctata tttccgttgg gacatcaaca    660
   ctaaaccaga gattggtacc aaaaatagct actagatcca aagtaaacgg gcaaagtgga    720
   aggatggatt tcttctggac aattttaaaa ccgaatgatg caatcaactt cgagagtaat    780
   ggaaatttca ttgctccaga atatgcatac aaaattgtca agaaagggga ctcagcaatt    840
   gttaaaagtg aagtggaata tggtaactgc aacacaaagt gtcaaactcc aataggggcg    900
   ataaactcta gtatgccatt ccacaacata caccctctca ccatcgggga atgccccaaa    960
   tatgtgaaat caaacaaatt agtccttgcg actgggctca gaaatagtcc tctaagagaa   1020
   agaagaagaa aaagaggact atttggagct atagcagggt ttatagaggg aggatggcag   1080
   ggaatggtag atggttggta tgggtaccac catagcaatg agcaggggag tgggtacgct 1140
   gcagacaaag aatccactca aaaggcaata gatggagtca ccaataaggt caactcgatc 1200
   attgacaaaa tgaacactca gtttgaggcc gttggaaggg aatttaataa cttagaaagg 1260
   agaatagaga atttaaacaa gaaaatggaa gacggattcc tagatgtctg gacttataat 1320
   gctgaacttc tggttctcat ggaaaatgag agaactctag acttccatga ttcaaatgtc 1380
   aagaaccttt acgacaaggt ccgactacag cttagggata atgcaaagga gctgggtaac 1440
   ggttgtttcg agttctatca caaatgtgat aatgaatgta tggaaagtgt aagaaacgga 1500
   acgtatgact acccgcagta ttcagaagaa gcaagattaa aaagagagga aataagtgga 1560
   gtaaaattgg aatcaatagg aacttaccaa atactgtcaa tttattcaac agttgcgagt 1620
   tctctagcac tggcaatcat ggtggctggt ctatctttgt ggatgtgctc caatgggtcg 1680
   ttacaatgca gaatttgcat ttaa 1704
<210> 5
   <211> 1701
   <212> DNA
   <213> Influenza A virus
<400> 5
   atgaaggcaa tactagtagt tctgctatat acatttgcaa ccgcaaatgc agacacatta     60
   tgtataggtt atcatgcgaa caattcaaca gacactgtag acacagtact agaaaagaat    120
   gtaacagtaa cacactctgt taaccttcta gaagacaagc ataacgggaa actatgcaaa    180
   ctaagagggg tagccccatt gcatttgggt aaatgtaaca ttgctggctg gatcctggga    240
   aatccagagt gtgaatcact ctccacagca agctcatggt cctacattgt ggaaacacct    300
   agttcagaca atggaacgtg ttacccagga gatttcatcg attatgagga gctaagagag    360
   caattgagct cagtgtcatc atttgaaagg tttgagatat tccccaagac aagttcatgg    420
   cccaatcatg actcgaacaa aggtgtaacg gcagcatgtc ctcatgctgg agcaaaaagc    480
   ttctacaaaa atttaatatg gctagttaaa aaaggaaatt catacccaaa gctcagcaaa    540
   tcctacatta atgataaagg gaaagaagtc ctcgtgctat ggggcattca ccatccatct    600
   actagtgctg accaacaaag tctctatcag aatgcagatg catatgtttt tgtggggtca    660
   tcaagataca gcaagaagtt caagccggaa atagcaataa gacccaaagt gagggrtcra     720
   gaagggagaa tgaactatta ctggacacta gtagagccgg gagacaaaat aacattcgaa     780
   gcaactggaa atctagtggt accgagatat gcattcgcaa tggaaagaaa tgctggatct     840
   ggtattatca tttcagatac accagtccac gattgcaata caacttgtca aacacccaag     900
   ggtgctataa acaccagcct cccatttcag aatatacatc cgatcacaat tggaaaatgt     960
   ccaaaatatg taaaaagcac aaaattgaga ctggccacag gattgaggaa tatcccgtct    1020
   attcaatcta gaggcctatt tggggccatt gccggtttca ttgaaggggg gtggacaggg    1080
   atggtagatg gatggtacgg ttatcaccat caaaatgagc aggggtcagg atatgcagcc    1140
   gacctgaaga gcacacagaa tgccattgac gagattacta acaaagtaaa ttctgttatt    1200
   gaaaagatga atacacagtt cacagcagta ggtaaagagt tcaaccacct ggaaaaaaga    1260
   atagagaatt taaataaaaa agttgatgat ggtttcctgg acatttggac ttacaatgcc    1320
   gaactgttgg ttctattgga aaatgaaaga actttggact accacgattc aaatgtgaag    1380
   aacttatatg aaaaggtaag aagccagcta aaaaacaatg ccaaggaaat tggaaacggc    1440
   tgctttgaat tttaccacaa atgcgataac acgtgcatgg aaagtgtcaa aaatgggact    1500
   tatgactacc caaaatactc agaggaagca aaattaaaca gagaagaaat agatggggta    1560
   aagctggaat caacaaggat ttaccagatt ttggcgatct attcaactgt cgccagttca    1620
   ttggtactgg tagtctccct gggggcaatc agtttctgga tgtgctctaa tgggtctcta    1680
   cagtgtagaa tatgtattta a                                              1701
<210> 6
   <211> 849
   <212> DNA
   <213> Vaccinia virus
<400> 6
   gatgatgatg atgatgatga tgatgatgat gtcatagacg atgatgatta taatccaaaa      60
   cccactccga taccggagcc tcaccctaga ccaccgtttc ccagacatga atatcataag     120
   aggccgaaag ttcttcctgt agaagaacct gatcctgtca aaaaagacgc ggatcgtata     180
   agacttgata atcatatatt aaacacattg gatcataatc ttaattccat cggacactat     240
   tgttgtgata cagcagcagt tgataggtta gaacatcaca ttgaaacatt gggacaatat     300
   gcagtaatac tagcaagaaa gataaatatg caaacattac tgttcccatg gccattacct     360
   actgtccatc cacatgcgat agatggtagt attccgccac atgggagatc tacgatctta     420
   taattacacg attgtagtta agttttgaat aaaatttttt tataataaat agaggtcacg     480
   aacctcgact ctagaggatc ccattgtgaa aaattgaaaa actagtctaa tttattgcac     540
   ggtgtgaaaa attgaaaaac tagtctaatt tattgcacgg tgtgaaaaat tgaaaaacta     600
   gtctaattta ttgcacggtg tgaaaaattg aaaaactagt ctaatttatt gcacggtgtg     660
   aaaaattgaa aaactagtct aatttattgc acggtgtgaa aaattgaaaa actagtctaa     720
   tttattgcac ggtgtgaaaa attgaaaaac tagtctaatt tattgcacgg tgtgaaaaat     780
   tgaaaaacta gtctaattta ttgcacggtg tgaaaaattg aaaaactagt taatttattg     840
   cacggtgtg                                                             849
<210> 7
   <211> 20
   <212> DNA
   <213> Artificial
<220>
   <223> Synthetic DNA
<400> 7
   cactcagttt gaggccgttg 20
<210> 8
   <211> 19
   <212> DNA
   <213> Artificial
<220>
   <223> Synthetic DNA
<400> 8
   aatgcgaact gttggttct 19
<210> 9
   <211> 21
   <212> DNA
   <213> Artificial
<220>
   <223> Synthetic DNA
<400> 9
   ctagttctga gaaaccagag g 21
<210> 10
   <211> 20
   <212> PRT
   <213> Influenza A virus
<400> 10
   Asn Asp Ala Ala Glu Gln Thr Lys Leu Tyr Gln Asn Pro Thr Thr Tyr
   1 5 10 15
   Ile Ser Val Gly
   20
<210> 11
   <211> 12
   <212> PRT
   <213> Influenza A virus
<400> 11
   Tyr Ser Lys Lys Phe Lys Pro Glu Ile Ala Ile Arg
   1 5 10
<210> 12
   <211> 1695
   <212> DNA
   <213> Artificial
<220>
   <223> Recombinant DNA
<400> 12
   atggagaaaa tagtgcttct ttttgcaata gtcagtcttg ttaaaagtga tcagatttgc 60
   attggttacc atgcaaacaa ctcgacagag caggttgaca caataatgga aaagaacgtt 120
   actgttacac atgcccaaga catactggaa aagacacaca atgggaagct ctgcgatcta 180
   gatggagtga agcctctaat tttgagagat tgtagtgtag ctggatggct cctcggaaac 240
   ccaatgtgtg acgaattcat caatgtgccg gaatggtctt acatagtgga gaaggccaat 300
   ccagtcaatg acctctgtta cccaggggat ttcaatgact atgaagaatt gaaacaccta 360
   ttgagcagaa taaaccattt tgagaaaatt cagatcatcc ccaaaagttc ttggtccagt 420
   catgaagcct cattgggggt gagctcagca tgtccatacc agggaaagtc ctcctttttc 480
   agaaatgtgg tatggcttat caaaaagaac agtacatacc caacaataaa gaggagctac 540
   aataatacca accaagaaga tcttttggta ctgtggggga ttcaccatcc taatgatgcg 600
   gcagagcaga caaagctcta tcaaaaccca accacctata tttccgttgg gacatctaca 660
   ctaaaccaga gattggtacc aagaatagct actagatcca aagtaaacgg gcaaagtgga 720
   aggatggagt tcttctggac aattttaaaa ccgaatgatg caatcaactt cgagagtaat 780
   ggaaatttca ttgctccaga atatgcatac aaaattgtca agaaagggga ctcaacaatt 840
   atgaaaagtg aattggaata tggtaactgc aacaccaagt gtcaaactcc aatgggggcg 900
   ataaactcta gcatgccatt ccacaatata caccctctca ccatcgggga atgccccaaa 960
   tatgtgaaat caaacagatt agtccttgcg actgggctca gaaatagccc tcaaagagag 1020
   acgagaggat tatttggagc tatagcaggt tttatagagg gaggatggca gggaatggta 1080
   gatggttggt atgggtacca ccatagcaac gagcagggga gtgggtacgc tgcagacaaa 1140
   gaatccactc aaaaggcaat agatggagtc accaataagg tcaactcgat tattgacaaa 1200
   atgaacactc agtttgaggc cgttggaagg gaatttaaca acttagaaag gagaatagag 1260
   aatttaaaca agaagatgga agacgggttc ctagatgtct ggacttataa tgctgaactt 1320
   ctagttctca tggaaaacga gagaactcta gactttcatg actcaaatgt caagaacctt 1380
   tacgacaagg tccgactaca gcttagggat aatgcaaagg agctgggtaa cggttgtttc 1440
   gagttctatc ataaatgtga taatgaatgt atggaaagtg taagaaacgg aacgtatgac 1500
   tacccgcagt attcagaaga agcaagacta aaaagagagg aaataagtgg agtaaaattg 1560
   gaatcaatag gaatttacca aatattgtca atttattcta cagtggcgag ctccctagca 1620
   ctggcaatca tggtagctgg tctatcctta tggatgtgct ccaatgggtc gttacaatgc 1680
   agaatttgca tttaa 1695
<210> 13
   <211> 1695
   <212> DNA
   <213> Artificial
<220>
   <223> Recombinant DNA
<400> 13
   atggagaaaa tagtgcttct tcttgcaata gtcagtcttg ttaaaagtga tcagatttgc 60
   attggttacc atgcaaacaa ttcaacagag caggttgaca caatcatgga aaagaacgtt 120
   actgttacac atgcccaaga catactggaa aagacacaca acgggaagct ctgcgatcta 180
   gatggagtga agcctctaat tttaagagat tgtagtgtag ctggatggct cctcgggaac 240
   ccaatgtgtg acgaattcat caatgtaccg gaatggtctt acatagtgga gaaggccaat 300
   ccaaccaatg acctctgtta cccagggagt ttcaacgact atgaagaact gaaacaccta 360
   ttgagcagaa taaaccattt tgagaaaatt caaatcatcc ccaaaagttc ttggtccgat 420
   catgaagcct catcaggagt gagctcagca tgtccatacc tgggaagtcc ctcctttttt 480
   agaaatgtgg tatggcttat caaaaagaac agtacatacc caacaataaa gaaaagctac 540
   aataatacca accaagaaga tcttttggta ctgtggggaa ttcaccatcc taatgatgcg 600
   gcagagcaga caaggctata tcaaaaccca accacctata tttccattgg gacatcaaca 660
   ctaaaccaga gattggtacc aaaaatagct actagatcca aagtaaacgg gcaaagtgga 720
   aggatggagt tcttctggac aattttaaaa cctaatgatg caatcaactt cgagagtaat 780
   ggaaatttca ttgctccaga atatgcatac aaaattgtca agaaagggga ctcagcaatt 840
   atgaaaagtg aattggaata tggtaactgc aacaccaagt gtcaaactcc aatgggggcg 900
   ataaactcta gtatgccatt ccacaacata caccctctca ccatcgggga atgccccaaa 960
   tatgtgaaat caaacagatt agtccttgca acagggctca gaaatagccc tcaaagagag 1020
   agcagaggac tatttggagc tatagcaggt tttatagagg gaggatggca gggaatggta 1080
   gatggttggt atgggtacca ccatagcaat gagcagggga gtgggtacgc tgcagacaaa 1140
   gaatccactc aaaaggcaat agatggagtc accaataagg tcaactcaat cattgacaaa 1200
   atgaacactc agtttgaggc cgttggaagg gaatttaata acttagaaag gagaatagag 1260
   aatttaaaca agaagatgga agacgggttt ctagatgtct ggacttataa tgccgaactt    1320
   ctggttctca tggaaaatga gagaactcta gactttcatg actcaaatgt taagaacctc    1380
   tacgacaagg tccgactaca gcttagggat aatgcaaagg agctgggtaa cggttgtttc    1440
   gagttctatc acaaatgtga taatgaatgt atggaaagta taagaaacgg aacgtacaac    1500
   tatccgcagt attcagaaga agcaagatta aaaagagagg aaataagtgg ggtaaaattg    1560
   gaatcaatag gaacttacca aatactgtca atttattcaa cagtggcgag ttccctagca    1620
   ctggcaatca tgatggctgg tctatcttta tggatgtgct ccaatggatc gttacaatgc    1680
   agaatttgca tttaa                                                    1695
<210> 14
   <211> 1692
   <212> DNA
   <213> Artificial
<220>
   <223> Recombinant DNA
<400> 14
   atggagaaaa tagtgcttct tcttgcaata gtcagccttg ttaaaagtga tcagatttgc    60
   attggttacc atgcaaacaa ctcgacagag caggttgaca caataatgga aaagaacgtt   120
   actgttacac atgcccaaga catactggaa aagacacaca acgggaagct ctgcgatcta   180
   gatggagtga agcctctgat tttaagagat tgtagtgtag ctggatggct cctcggaaac   240
   ccaatgtgtg acgaattcat caatgtgccg gaatggtctt acatagtgga gaaggccaac   300
   ccagccaatg acctctgtta cccagggaat ttcaacgact atgaagaact gaaacaccta   360
   ttgagcagaa taaaccattt tgagaaaatt cagatcatcc ccaaaagttc ttggtccgat   420
   catgaagcct catcaggggt gagctcagca tgtccatacc agggaacgcc ctcctttttc   480
   agaaatgtgg tatggcttat caaaaagaac aatacatacc caacaataaa gagaagctac   540
   aataatacca accaggaaga tcttttgata ctgtggggga ttcatcattc taatgatgcg   600
   gcagagcaga caaagctcta tcaaaaccca accacctata tttccgttgg gacatcaaca   660
   ctaaaccaga gattggtacc aaaaatagct actagatcca aagtaaacgg gcaaagtgga   720
   aggatggatt tcttctggac aattttaaaa ccgaatgatg caatcaactt cgagagtaat   780
   ggaaatttca ttgctccaga atatgcatac aaaattgtca agaaagggga ctcagcaatt 840
   gttaaaagtg aagtggaata tggtaactgc aacacaaagt gtcaaactcc aataggggcg 900
   ataaactcta gtatgccatt ccacaacata caccctctca ccatcgggga atgccccaaa 960
   tatgtgaaat caaacaaatt agtccttgcg actgggctca gaaatagtcc tctaagagaa 1020
   agaggactat ttggagctat agcagggttt atagagggag gatggcaggg aatggtagat 1080
   ggttggtatg ggtaccacca tagcaatgag caggggagtg ggtacgctgc agacaaagaa 1140
   tccactcaaa aggcaataga tggagtcacc aataaggtca actcgatcat tgacaaaatg 1200
   aacactcagt ttgaggccgt tggaagggaa tttaataact tagaaaggag aatagagaat 1260
   ttaaacaaga aaatggaaga cggattccta gatgtctgga cttataatgc tgaacttctg 1320
   gttctcatgg aaaatgagag aactctagac ttccatgatt caaatgtcaa gaacctttac 1380
   gacaaggtcc gactacagct tagggataat gcaaaggagc tgggtaacgg ttgtttcgag 1440
   ttctatcaca aatgtgataa tgaatgtatg gaaagtgtaa gaaacggaac gtatgactac 1500
   ccgcagtatt cagaagaagc aagattaaaa agagaggaaa taagtggagt aaaattggaa 1560
   tcaataggaa cttaccaaat actgtcaatt tattcaacag ttgcgagttc tctagcactg 1620
   gcaatcatgg tggctggtct atctttgtgg atgtgctcca atgggtcgtt acaatgcaga 1680
   atttgcattt aa 1692
<210> 15
   <211> 1701
   <212> DNA
   <213> Artificial
<220>
   <223> Recombinant DNA
<400> 15
   atgaaggcaa tactagtagt tctgctatat acatttgcaa ccgcaaatgc agacacatta 60
   tgtataggtt atcatgcgaa caattcaaca gacactgtag acacagtact agaaaagaat 120
   gtaacagtaa cacactctgt taaccttcta gaagacaagc ataacgggaa actatgcaaa 180
   ctaagagggg tagccccatt gcatttgggt aaatgtaaca ttgctggctg gatcctggga 240
   aatccagagt gtgaatcact ctccacagca agctcatggt cctacattgt ggaaacacct 300
   agttcagaca atggaacgtg ttacccagga gatttcatcg attatgagga gctaagagag 360
   caattgagct cagtgtcatc atttgaaagg tttgagatat tccccaagac aagttcatgg 420
   cccaatcatg actcgaacaa aggtgtaacg gcagcatgtc ctcatgctgg agcaaaaagc 480
   ttctacaaaa atttaatatg gctagttaaa aaaggaaatt catacccaaa gctcagcaaa 540
   tcctacatta atgataaagg gaaagaagtc ctcgtgctat ggggcattca ccatccatct 600
   actagtgctg accaacaaag tctctatcag aatgcagatg catatgtatt cgtggggtca 660
   tcaagataca gcaagaagtt caagccggaa atagcaataa gacccaaagt gagggatcga 720
   gaagggagaa tgaactatta ctggacacta gtagagccgg gagacaaaat aacattcgaa 780
   gcaactggaa atctagtggt accgagatat gcattcgcaa tggaaagaaa tgctggatct 840
   ggtattatca tttcagatac accagtccac gattgcaata caacttgtca aacacccaag 900
   ggtgctataa acaccagcct cccatttcag aatatacatc cgatcacaat tggaaaatgt 960
   ccaaaatatg taaaaagcac aaaattgaga ctggccacag gattgaggaa tatcccgtct 1020
   attcaatcta gaggcctatt tggggccatt gccggtttca ttgaaggggg gtggacaggg 1080
   atggtagatg gatggtacgg ttatcaccat caaaatgagc aggggtcagg atatgcagcc 1140
   gacctgaaga gcacacagaa tgccattgac gagattacta acaaagtaaa ttctgttatt 1200
   gaaaagatga atacacagtt cacagcagta ggtaaagagt tcaaccacct ggaaaaaaga 1260
   atagagaatt taaataaaaa agttgatgat ggtttcctgg acatttggac ttacaatgcc 1320
   gaactgttgg ttctattgga aaatgaaaga actttggact accacgattc aaatgtgaag 1380
   aacttatatg aaaaggtaag aagccagcta aaaaacaatg ccaaggaaat tggaaacggc 1440
   tgctttgaat tttaccacaa atgcgataac acgtgcatgg aaagtgtcaa aaatgggact 1500
   tatgactacc caaaatactc agaggaagca aaattaaaca gagaagaaat agatggggta 1560
   aagctggaat caacaaggat ttaccagatt ttggcgatct attcaactgt cgccagttca 1620
   ttggtactgg tagtctccct gggggcaatc agtttctgga tgtgctctaa tgggtctcta 1680
   cagtgtagaa tatgtattta a 1701